# EUROPEAN PATENT SPECIFICATION

(11) **EP 4 355 215 B1**
(45) Date of publication and mention of the grant of the patent: **14.01.2026**
(21) Application number: 22824396.0
(22) Date of filing: 13.06.2022
(51) Int. Cl.: A61B 5/12, A61B 5/024, A61B 5/24, A61B 5/00, H04R 25/00, A61B 5/0205

(54) **COCHLEA HEALTH MONITORING**
COCHLEA-GESUNDHEITSÜBERWACHUNG
SURVEILLANCE DE L'ÉTAT DE SANTÉ DE LA COCHLÉE

(30) Priority: 18.06.2021 US 202163212307 P
(43) Date of publication of application: 24.04.2024
(73) Proprietor: Cochlear Limited, Macquarie University, New South Wales 2109 (AU)
(72) Inventor: HEASMAN, John Michael, Macquarie University, New South Wales 2109 (AU); GIBSON, Peter, Macquarie University, New South Wales 2109 (AU)
(74) Representative: Grünecker Patent- und Rechtsanwälte PartG mbB
(86) International application number: PCT/IB2022/055440
(87) International publication number: WO 2022/263992

(56) References cited:
- WO-A1-2020/257367
- WO-A1-2021/081412
- WO-A1-2021/081412
- KR-B1- 101 754 840
- US-A1- 2008 188 762
- US-A1- 2011 009 712
- US-A1- 2017 360 364
- US-A1- 2017 360 364

## Description

### BACKGROUND

### Field of the Invention

The present subject matter relates generally to hearing prostheses.

### Related Art

US 2011/009712 A1 discloses implantable systems for monitoring for an impending myocardial infarction, which obtain a photoplethysmography signal or an impedance plethysmography signal indicative of changes in arterial blood volume.
US 2017/360364 A1 discloses in-situ techniques for monitoring a recipient's cochlea health to proactively identify changes to the recipient's cochlea health outside of a clinical setting.
WO 2021/081412 A1 discloses a system including a stimulation device configured to apply stimulation to a recipient, a sensing device configured to detect a physiological condition of the recipient, and a processing unit communicatively coupled to the stimulation device and the sensing device.
Medical devices have provided a wide range of therapeutic benefits to recipients over recent decades. Medical devices can include internal or implantable components/devices, external or wearable components/devices, or combinations thereof (e.g., a device having an external component communicating with an implantable component). Medical devices, such as traditional hearing aids, partially or fully-implantable hearing prostheses (e.g., bone conduction devices, mechanical stimulators, cochlear implants, *etc.),* pacemakers, defibrillators, functional electrical stimulation devices, and other medical devices, have been successful in performing lifesaving and/or lifestyle enhancement functions and/or recipient monitoring for a number of years.

The types of medical devices and the ranges of functions performed thereby have increased over the years. For example, many medical devices, sometimes referred to as "implantable medical devices," now often include one or more instruments, apparatus, sensors, processors, controllers or other functional mechanical or electrical components that are permanently or temporarily implanted in a recipient. These functional devices are typically used to diagnose, prevent, monitor, treat, or manage a disease/injury or symptom thereof, or to investigate, replace or modify the anatomy or a physiological process. Many of these functional devices utilize power and/or data received from external devices that are part of, or operate in conjunction with, implantable components.

### SUMMARY

An implantable medical device according to the invention includes the features defined in claim 1.

A non-transitory computer readable storage medium according to the invention includes the features defined in claim 14.

Embodiments include the features defined in the dependent claims.

### BRIEF DESCRIPTION OF THE DRAWINGS

Embodiments are described herein in conjunction with the accompanying drawings, in which:
FIG. 1A is a schematic diagram of an electro-acoustic hearing prosthesis in accordance with embodiments presented herein;
FIG. 1B is a block diagram of the electro-acoustic hearing prosthesis of FIG. 1A;
FIG. 1C is a schematic diagram illustrating further details of the electro-acoustic hearing prosthesis of FIG. 1A;
FIG. 1D is a block diagram of an alternative embodiment of an electro-acoustic hearing prosthesis;
FIG. 1E is a block diagram of another alternative embodiment of an electro-acoustic hearing prosthesis;
FIG. 2 is a diagram illustrating cochlea health analysis techniques in accordance with embodiments presented herein;
FIG. 3 is a block diagram of a cochlea health analysis module in accordance with embodiments presented herein;
FIG. 4 is a flowchart of a method in accordance with embodiments presented herein;
FIG. 5 is a schematic diagram of an illustrative cochlear implant system configured to implement the cochlea health monitoring techniques presented herein; and
FIG. 6 illustrates an example vestibular stimulator system, in accordance with certain embodiments presented herein.

### DETAILED DESCRIPTION

Auditory/hearing prosthesis recipients suffer from different types of hearing loss (e.g., conductive and/or sensorineural) and/or different degrees/severity of hearing loss. However, it is now common for many hearing prosthesis recipients to retain some residual natural hearing ability (residual hearing) after receiving the hearing prosthesis. That is, hearing prosthesis recipients often retain at least some of their natural ability to hear sounds without the aid of their hearing prosthesis. There has been increased focus on preserving a recipient's hearing during implantation of a hearing prosthesis into a recipient. For example, in the case of cochlear implants, progressive improvements in the design of intra-cochlear electrode arrays (stimulating assemblies), surgical implantation techniques, tooling, *etc.* have enabled atraumatic surgeries which preserve at least some of the recipient's fine inner ear structures (e.g., cochlea hair cells) and the natural cochlea function, particularly in the higher frequency regions of the cochlea. Accordingly, greater numbers of recipients have post-implantation residual hearing in an ipsilateral and/or contralateral ear. The benefits of post-implantation residual hearing may include, for example, improved sound localization, music appreciation, binaural release from unmasking, head shadow, and the ability to distinguish acoustic signals in a noisy environment.

However, there is also risk that a recipient's residual hearing, and more generally a recipient's cochlea health, can change or deteriorate post-implantation (i.e., after the hearing prosthesis has been implanted within the recipient). These changes may be related to, for example, inner ear inflammatory responses, fibrosis, ossification, auditory neuropathy, endolymphatic hydrops, electro-acoustic interaction/masking, and excitotoxicity. For example, a hearing prosthesis that is improperly configured for (i.e., improperly "fit" to) a recipient may result in the delivery of stimulation (electrical stimulation and/or acoustic stimulation) in a manner that causes acute and/or chronic losses in the recipient's residual hearing and/or in a manner that interferes with the transduction of information via the residual hearing structures.

In certain cases, losses to a recipient's residual hearing could render the recipient's hearing prostheses ineffective. In other cases, continued operation of the hearing prostheses could exacerbate the residual hearing loss. Presently, residual hearing loss and other changes to a recipient's cochlea health are only detected/identified within a clinical environment, typically using complex equipment and techniques implemented by trained audiologists/clinicians. However, recipients generally do not visit clinics on a regular basis due to, for example, costs, low availability of trained audiologists, such as in rural areas, *etc.* Therefore, the need to visit a clinic in order to check residual hearing and/or other cochlea health changes may not only be cost prohibitive for certain recipients, but may also result in the passage of a significant period of time before the residual hearing or other cochlea health change is identified, let alone addressed. Accordingly, conventional techniques are designed to detect residual hearing and/or other cochlea health changes only after such changes have occurred. That is, conventional techniques can only operate to retroactively address residual hearing and/or other cochlea health changes.

Presented herein are in-situ techniques for monitoring a recipient's cochlea health to proactively identify (i.e., predict) changes to the recipient's cochlea health outside of a clinical setting. As used herein, changes to a recipient's "cochlea health" includes changes in the recipient's residual hearing, such as changes to the function of the cochlea hair cells, synapses, spiral ganglions, dendrites, peripheral central processes, as well as other changes in the functioning of the cochlea/ inner ear. As described further below, the cochlea health monitoring techniques presented herein obtain one or more cochlea health biomarkers associated with a recipient's cochlea health, such as the recipient's residual hearing, and analyze these biomarkers to predict that a cochlea health change, sometimes referred to herein as an "inner ear crises," is likely to occur. Also as described further below, the techniques presented herein can be carried out repeatedly with minimal or no involvement, or possibly even awareness, by the recipient, and may be used to proactively remediate (e.g., prevent) changes to a recipient's cochlea health (e.g., ensure the stimulus is safe and to ensure on-going clinical utility).

For ease of illustration, embodiments are primarily described herein with reference to one type of implantable auditory/hearing prosthesis, namely an implantable electro-acoustic hearing prosthesis that is configured to deliver both electrical stimulation (i.e., electrical stimulation signals) and acoustic stimulation (i.e., acoustic stimulation signals) to a recipient. Acoustic stimulation combined with electrical stimulation is sometimes referred to herein as electro-acoustic stimulation. However, it is to be appreciated that the techniques presented herein may be used with a variety of other implantable medical device systems. For example, the techniques presented herein may be used with other hearing devices/prostheses, including combinations of any of a cochlear implant, middle ear auditory prosthesis (middle ear implant), bone conduction device, direct acoustic stimulator, electro-acoustic prosthesis, auditory brain stimulators, *etc.* The techniques presented herein may also be used with systems that comprise or include tinnitus therapy devices or vestibular devices.

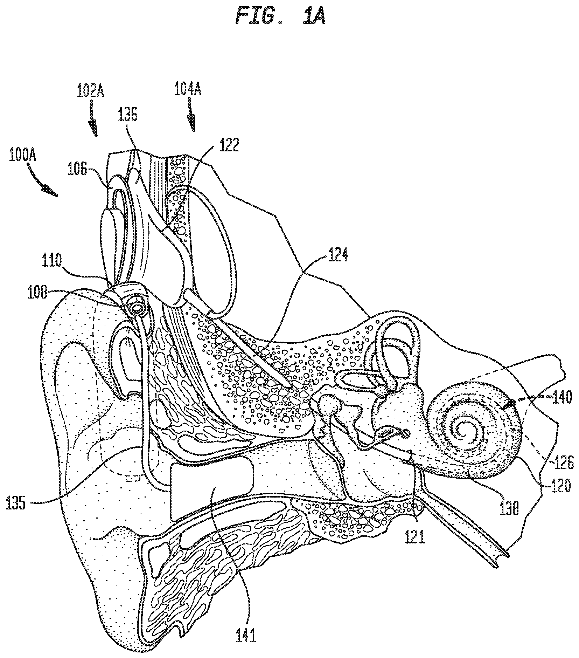

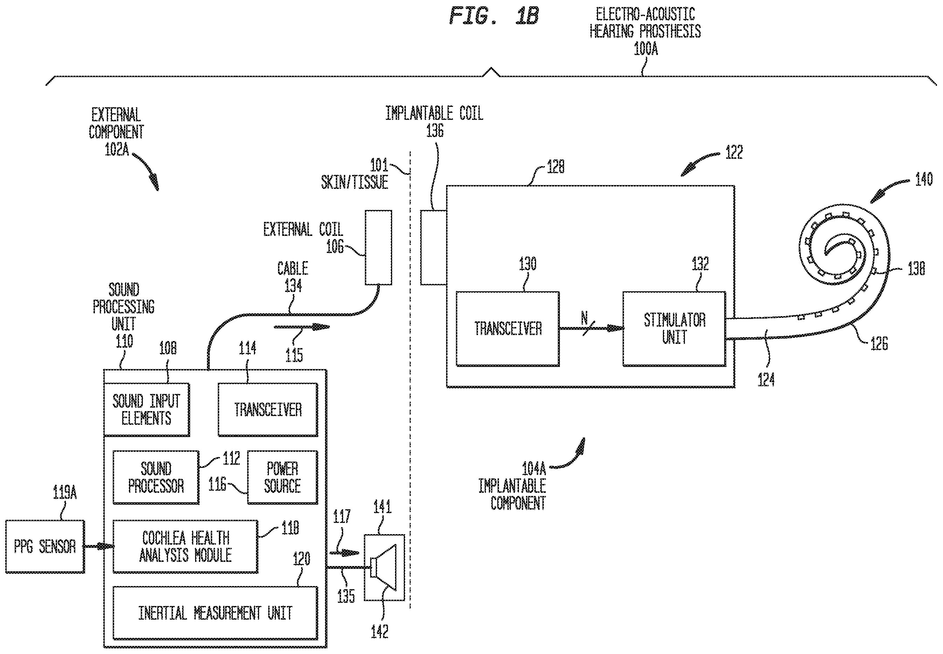

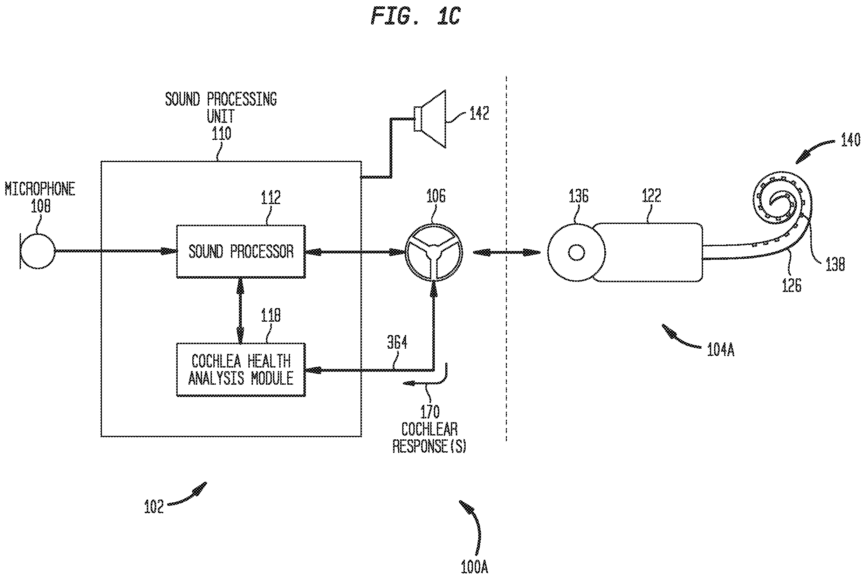

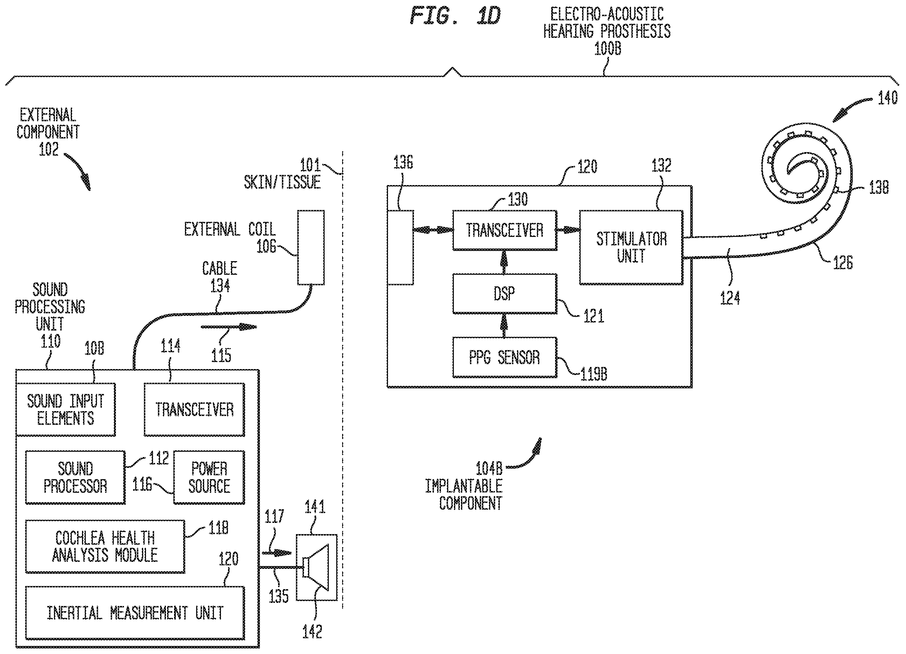

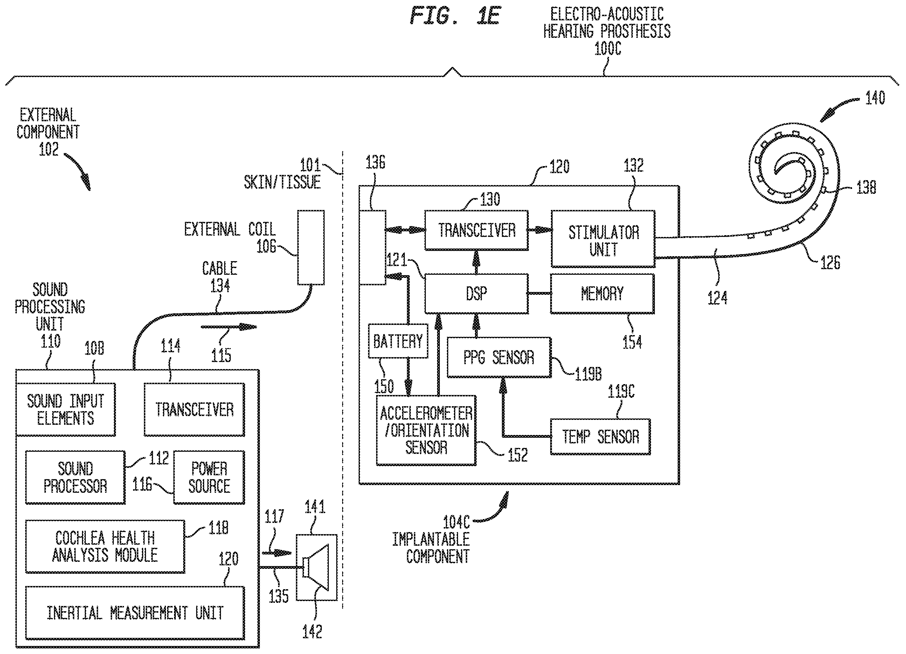

FIGs. 1A, 1B, 1C, 1D, and 1E are diagrams of an illustrative implantable electro-acoustic hearing prosthesis 100A configured to implement the cochlea health monitoring techniques presented herein. More specifically, FIGs. 1A, 1B, and 1C illustrate an electro-acoustic hearing prosthesis 100A that includes an external component 102A and an internal/implantable component 104A. The external component 102A is configured to be directly or indirectly attached to the body of a recipient, while the implantable component 104A is configured to be subcutaneously implanted within the recipient (i.e., under the skin/tissue 101 of the recipient). FIG. 1C is a schematic diagram illustrating further details of the external component 102A, namely operation of the cochlea health analysis module 118.

The external component 102A comprises an external coil 106 and a sound processing unit 110 connected via, for example, a cable 134. The external coil 106 is typically a wire antenna coil comprised of multiple turns of electrically insulated single-strand or multi-strand platinum or gold wire. Generally, a magnet (not shown in FIG. 1A) is fixed relative to the external coil 106. The external component 102A also comprises a hearing aid component 141 that includes a receiver 142 (FIG. 1B). The hearing aid component 141 is connected to the sound processing unit 110 via a cable 135. The receiver 142 is a component that is configured to deliver an acoustic signal (acoustic stimulation) to the recipient via the recipient's ear canal and middle ear. The receiver 142 may be, for example, positioned in or near the recipient's outer ear.

The sound processing unit 110 comprises one or more sound input elements, such as microphones 108, a sound processor 112, an external transceiver unit (transceiver) 114, a power source 116, a cochlea health analysis module 118, and an inertial measurement unit ("IMU") 120. The sound processing unit 110 may be, for example, a behind-the-ear ("BTE") sound processing unit or other type of processing unit worn on the recipient's head.

The implantable component 104A comprises an implant body (main module) 122, a lead region 124, and an elongate intra-cochlear stimulating assembly (electrode array) 126. The implant body 122 generally comprises a hermetically-sealed housing 128 in which an internal transceiver unit (transceiver) 130 and a stimulator unit 132 are disposed. The implant body 122 also includes an internal/implantable coil 136 that is generally external to the housing 128, but which is connected to the transceiver 130 via a hermetic feedthrough (not shown). Implantable coil 136 is typically a wire antenna coil comprised of multiple turns of electrically insulated single-strand or multi-strand platinum or gold wire. The electrical insulation of implantable coil 136 is provided by a flexible molding (e.g., silicone molding), which is not shown in FIG. 1B. Generally, a magnet (not shown in FIG. 1B) is fixed relative to the implantable coil 136.

Elongate stimulating assembly 126 is configured to be at least partially implanted in the recipient's cochlea (not shown in FIG. 1B) and includes a plurality of longitudinally spaced intra-cochlear stimulating contacts (e.g., electrical and/or optical contacts) 138 that collectively form a contact array 140. Stimulating assembly 126 extends through an opening in the cochlea (e.g., cochleostomy, the round window, *etc.)* and has a proximal end connected to stimulator unit 132 via lead region 124 and a hermetic feedthrough (not shown in FIG. 1B). As such, lead region 124 couples the stimulating assembly 126 to the implant body 122 and, more particularly, stimulator unit 132.

Returning to external component 102A, the microphone(s) 108 and/or other sound input elements (not shown) are configured to detect/receive sound signals and generate electrical output signals therefrom. These output signals are representative of the detected sound signals. In addition to the one or more microphones 108, the sound processing unit 110 may include other types of sound input elements (e.g., telecoils, audio inputs, *etc.)* to receive sound signals. However, merely for ease of illustration, these other types of sound input elements have been omitted from FIG. 1B.

The sound processing unit 110 includes the inertial measurement unit 120. The inertial measurement unit 120 is configured to measure the inertia of the recipient's head, that is, motion of the recipient's head. As such, inertial measurement unit 120 comprises one or more sensors 125 each configured to sense one or more of rectilinear or rotatory motion in the same or different axes. Examples of sensors 125 that may be used as part of inertial measurement unit 120 include accelerometers, gyroscopes, compasses, and the like. Such sensors may be implemented in, for example, micro electromechanical systems (MEMS) or with other technology suitable for the particular application.

The sound processor 112 is configured to execute sound processing and coding to convert the output signals received from the sound input elements into coded data signals that represent acoustic and/or electrical stimulation for delivery to the recipient. That is, as noted, the electro-acoustic hearing prosthesis 100A operates to evoke perception by the recipient of sound signals received by the sound input elements (e.g., microphones 108) through the delivery of one or both of electrical stimulation signals and acoustic stimulation signals to the recipient. As such, depending on a variety of factors, the sound processor 112 is configured to convert the output signals received from the sound input elements into a first set of coded signals representative of electrical stimulation and/or into a second set of coded signals representative of acoustic stimulation. The coded signals representative of electrical stimulation are represented in FIG. 1B by arrow 115, while the coded signals representative of acoustic stimulation are represented in FIG. 1B by arrow 117.

The coded signals 115 are provided to the transceiver 114. The transceiver 114 is configured to transcutaneously transfer the coded signals 115 to the implantable component 104A via external coil 106. More specifically, the magnets fixed relative to the external coil 106 and the implantable coil 136 facilitate the operational alignment of the external coil 106 with the implantable coil 136. This operational alignment of the coils enables the external coil 106 to transmit the coded data signals 115, as well as power signals received from power source 116, to the implantable coil 136. In certain examples, external coil 106 transmits the signals to implantable coil 136 via a radio frequency (RF) link. However, various other types of energy transfer, such as infrared (IR), electromagnetic, capacitive and inductive transfer, may be used to transfer the power and/or data from an external component to an electro-acoustic hearing prosthesis and, as such, FIG. 1B illustrates only one example arrangement.

In general, the coded data and power signals are received at the transceiver 130 and provided to the stimulator unit 132. The stimulator unit 132 is configured to utilize the coded data signals 115 to generate stimulation signals (e.g., current signals) for delivery to the recipient's cochlea via one or more stimulating contacts 138. In this way, electro-acoustic hearing prosthesis 100A stimulates the recipient's auditory nerve cells, bypassing absent or defective hair cells that normally transduce acoustic vibrations into neural activity, in a manner that causes the recipient to perceive the received sound signals.

As noted above, it is common for hearing prosthesis recipients to retain at least part of this normal hearing functionality (i.e., retain at least one residual hearing). Therefore, the cochlea of a hearing prosthesis recipient can be acoustically stimulated upon delivery of a sound signal to the recipient's outer ear, with the aid of the hearing prosthesis itself or, in certain cases, without the aid of the hearing prosthesis. In the example of FIGs. 1A and 1B, the receiver 142 is used to aid the recipient's residual hearing. More specifically, the coded signals 117 (i.e., the signals representative of acoustic stimulation) are provided to the receiver 142. The receiver 142 is configured to utilize the coded signals 117 to generate the acoustic stimulation signals that are provided to the recipient. In other words, the receiver 142 is used to enhance, and/or amplify a sound signal which is delivered to the cochlea via the middle ear bones and oval window, thereby creating dynamic pressure changes in the perilymph within the cochlea.

As noted above, a recipient's cochlea health (e.g., residual hearing, neural survival, *etc.),* can change post-implantation. Therefore, in accordance with embodiments presented herein, the electro-acoustic hearing prosthesis 100A comprises the cochlea health analysis module 118 that is configured to proactively identify (i.e., predict) post-implantation changes to a recipient's cochlea health and, potentially, initiate one or more remedial actions to, for example, prevent significant changes to the recipient's cochlea health. More specifically, the cochlea health analysis module 118 is configured to measure, determine, or otherwise obtain one or more biomarkers associated with the recipient's cochlea health. For example, the cochlea health analysis module 118 is configured to obtain sensor data from at least one PPG sensor 119A. In the embodiment of FIG. 1B, the at least one PPG sensor 119A is positioned on the recipient's body, and provides a biomarker indicating one or more of a heart rate, cardiac cycle, respiration, blood pressure, or blood oxygenation level of the recipient. Some recipients have two cochlear implants (e.g., one in each ear), and thus, in some embodiments, biomarkers from more than one PPG sensor 119A can provide the indications above.

In some embodiments, input(s) received from a PPG sensor integrated with an implantable component (e.g., cochlear implant) are augmented by additional data provided by other PPG sensors, for example, those included in wearable devices attached at various locations of the recipient, such as at a wrist of the recipient (e.g., smartwatches, fitness trackers, etc.). Differences in PPG sensor measurements made by a PPG sensor integrated with an implantable component and a separate PPG sensor in a different location can identify differences in blood flow between the two locations, be used to identify a delay in a blood pressure wave, etc. For example, under certain conditions, such as when an artery of the heart is beginning to experience a blockage, the blockage may be detectable via changes in timing between blood pressure waves at the two or more locations.

The cochlea health analysis module 118 is then configured to analyze one or more of these biomarkers to detect/identify one or more precursors of a change to the cochlea health (i.e., inner ear crises), such as precursors to residual hearing loss. Thus, in some embodiments, a sensor placed outside the inner ear (e.g., a PPG sensor placed on a body of the recipient) provides input useful, in combination with input from other sensors positioned within the inner ear or in other parts of the body (e.g., via a wearable device), in assessing cochlea health.

As used herein, "precursors" to cochlea health changes are detectable events corresponding to patterns of behavior or established patterns relating to known pathologies, pathophysiology or physiology that correlate to potential future changes in the cochlea health, such as potential loss of residual hearing (i.e., predetermined risk factors for residual hearing loss) and or trauma to inner ear biological structures.

In one form, the precursors to cochlea health include inflammation or cardiovascular condition(s) that are detectable via the PPG sensor 119A and/or individual changes in one or more of a heart rate, cardiac cycle, a respiration rate, blood pressure, or blood oxygenation level of the recipient. Precursors also include, in some embodiments, electro-acoustic phenomena within the recipient's cochlea that are suggestive of potential residual hearing loss (e.g., significant residual hearing loss, residual hearing loss that is noticeable/perceivable by the recipient, *etc.).* As used herein, "electro-acoustic phenomena" are interactions of electrical and acoustic stimulation, or effects thereof, that can be detected and correlated to patterns of behavior or established patterns relating to known pathologies, pathophysiology or physiology associated with residual hearing loss. Some embodiments detect trauma experienced by the recipient, for example, via an accelerometer. Measurements collected from an accelerometer included in one or more of the cochlear implant or sound processor detect, in some embodiments, an impact to a head of the recipient. The impact can, in some circumstances, affect blood supply resulting directly from the impact itself. Alternatively, the impact can result in a reduction in mobility of the recipient, which can also, in some circumstances, result in a change in blood supply.

Based on the analysis of the biomarkers and the identification of one or more cochlea health change precursors, the cochlea health analysis module 118 can initiate one or more actions (treatments) to remediate the cochlea health changes. In some embodiments, the cochlea health analysis module 118 is configured to generate an alert (e.g., to a clinician or primary health care professional) indicating any identified cochlea health change precursors and/or any recommended remediation procedures. The alert is generated, in at least some embodiments, via any messaging technology such as email, text, pager, or other message technology.

In one example, the cochlea health analysis module 118 operates as a closed-loop element that is configured to auto-regulate, for example, the delivered stimulation (acoustic and electric stimulation signal properties/parameters) in order to minimize the likelihood of damaging or masking the physiological structures supporting residual hearing (e.g., a real-time control / regulatory system to minimize the loss of residual hearing post-implantation). Without regulation of the delivered stimulation, there is a risk that the post-implantation residual hearing may be compromised by the inadequately configured stimulation. For example, either the electrical and acoustic stimulation in combination (electro-acoustic) or in isolation (electric or acoustic) could be improperly clinically configured so as to cause either acute or chronic changes (injurious) to the cochlea health or, to interfere with the transduction of information via the residual acoustic hearing structures (e.g., by electroacoustic interactions including masking).

Alternatively, changes to a recipient's health may arise after implantation that are indicative of changes to the recipient's cochlea health. For example, cardiovascular conditions indicated by the PPG sensor 119A, in some cases, indicate blood flow to the cochlea and thereby impact a long-term health of the organ. Hypertension detectable by the PPG sensor 119A, in some circumstances, leads to hearing impairment. Heart rate variability detected by the PPG sensor 119A indicates, in some circumstances, a systemic infection that risks propagation to the cochlea or vestibular system. Such an infection risks permanent loss of either residual acoustic hearing or neural structures associated with electrical hearing. A drop in blood pressure that is detected via the PPG sensor 119A is indicative, in some circumstances, of a dysfunctional vestibular system.

The closed-loop features of the embodiments presented herein allow a hearing prosthesis to constantly adapt and update stimulation based on the measured biomarkers. This feature can be used to efficiently respond to, for example, a transient change to the cochlea due to an acute inflammatory response, or a chronic inflammatory process that may potentially induce permanent loss of hearing or progressive loss in residual hearing. That is, a hearing prosthesis in accordance with embodiments presented herein may employ the biomarkers as real-time feedback for regulation of the stimulation signals which originate from the hearing prosthesis to provide for the long-term preservation of the recipient's cochlea health, including residual hearing. In some embodiments, the biomarkers are used by the hearing prosthesis to regulate delivery of therapeutic substances (pharmaceutical agents) from an active delivery system.

FIG. 1D illustrates an alternative embodiment of an electro-acoustic hearing prosthesis, referred to as electro-acoustic hearing prosthesis 100B. Whereas the hearing prosthesis 100A of FIGs. 1A and 1B is configured to provide a PPG sensor that is external and attached to a body of the recipient, the embodiment of the hearing prosthesis 100B of FIG. 1D provides at least one implantable PPG sensor 119B within an implantable component 104B. The implantable component 104B of FIG. 1D also includes a digital signal processor (DSP) 121. The DSP 121 is configured to receive data from the at least one PPG sensor 119B and provide the data to the cochlea health analysis module 118 via the transceiver 130 and internal coil 136. In some embodiments, the at least one PPG sensor 119B is implanted in an ear canal of the recipient. For example, the PPG sensor 119B is implanted, in various embodiments, in a canal of the inner ear, an internal auditory canal, or an external auditory canal. In some other embodiments, the at least one PPG sensor 119B is implanted proximate to a blood-labyrinthine barrier (BLB) of the cochlea.

FIG. 1D also shows that the implantable component 104B includes a temperature sensor 119C where, in certain embodiments, temperature measurements made by the temperature sensor 119C are provided to the DSP 121. In certain such embodiments, the DSP 121 utilizes measurements received from the PPG sensor 119B and/or temperature sensor 119C to detect a variety of health conditions of the recipient of the electro-acoustic hearing prosthesis 100B. For example, as discussed below, measurements received from the PPG sensor 119B and/or temperature sensor 119C are used to detect infection of a middle ear of the recipient, or other health conditions.

In some embodiments, the DSP 121 is configured to detect a respiration rate and/or respiration depth based on input from the PPG sensor 119B. Respiration rate and/or respiration depth are factors in blood oxygen levels. Thus, in some embodiments, the implantable component 104B is able to detect health conditions relating to respiration rate and/or respiration depth, and the electro-acoustic hearing prosthesis 100B can generate an alert if the respiration rate and/or respiration depth transgress one or more predefined thresholds.

While the implantable component 104B is shown in FIG. 1D proximate to the external component 106, in some embodiments, the implantable component 104B receives power from an alternative power source, such as a pillow charger. The implantable component 104B is configured, in some embodiments, to operate in a low power mode when receiving power from such a power source, and to store information collected while in the low power mode for later uploading to the external component 102 upon being reconnected. For example, the DSP 121, in some embodiments, operates in a low power mode to collect information from the PPG sensor 119B and/or temperature sensor 119C for later upload to the external component 102.

FIG. 1E illustrates an alternative embodiment of an electro-acoustic hearing prosthesis, referred to as electro-acoustic hearing prosthesis 100C. The implantable component 104C of the electro-acoustic hearing prosthesis 100C varies from the implantable component 104B of the electro-acoustic hearing prosthesis 100B of FIG. 1D in that the implantable component 104C is equipped with a battery 150. The battery 150 is charged via the coil 136 and provides power to operate an accelerometer/orientation sensor 152 when the external component 102 is not present (for example, when the recipient is sleeping).

The battery 150 is also configured to operate the DSP 121 (e.g., in a low power mode) when the external component 102 is not present. The DSP 121 is configured, in some embodiments, to detect an orientation of the recipient via measurements received from the accelerometer/orientation sensor 152. For example, the DSP 121 determines, in some embodiments, a recipient is asleep or in a sleeping configuration if the orientation of the recipient is detected to be horizontal for a period of time (and other movements of the recipient are relatively limited). Some embodiments condition a low power mode of operation of the implantable component 104C based, in part, on a detection that the recipient is asleep or in a sleeping configuration.

In some embodiments, the DSP 121 collects measurements from the PPG sensor 119B and/or accelerometer/orientation sensor 152 in a low power mode based on power provided by the battery 150. Alternatively, embodiments of the implantable component 104B receive power from an alternative power source, such as a pillow charger, and perform these functions, and those discussed below, without use of a battery 150.

Some embodiments of the implantable component 104C are equipped with a memory 154 as shown. In some embodiments, the DSP 121 is configured to receive measurements from the PPG sensor 119B and/or the temperature sensor 119C and store the measurements in the memory 154 for later retrieval, for example, when the sound processor or eternal component 102 is again in communication with the implantable component 104C. By providing limited operation of the electro-acoustic hearing prosthesis 100C when the external component 102 is not present, collection of some health information is possible during both day and night time periods, as many recipients prefer to remove their sound processor (e.g., analogous to the external component 102) when sleeping. Thus, the embodiment of the implantable component 104C allows for monitoring of health information during both a day time environment as well as a night time environment when the recipient is asleep.

Some embodiments condition detection of abnormal health conditions based in part, on whether the recipient is determined to be asleep or awake. For example, a nominal level of blood flow or an exceptional level of blood flow indicated by input from the PPG sensor 119 is based, in some embodiments, on whether the recipient is detected to be asleep or awake. Thus, in some embodiments, the DSP 121 is configured to store only those indications that are classified as exceptions, in order to save space in the memory 154. Thus, different thresholds are compared to measurements received from the PPG sensor 119B, in some embodiments, based on whether the recipient is determined to be awake or asleep.

In some embodiments, the implantable component 104C is configured to operate in a low power mode and receive power via the coil 136 while a recipient is asleep or in a sleep configuration (e.g., w/o a sound processor / external component 102). In some of these embodiments, the battery may be omitted with the accelerometer/orientation sensor 152, DSP 121, memory 154, PPG sensor 119B and/or temperature sensor 119C operating based on power received from a pillow charger (or other external device that conveys power to the implantable component 104 when the recipient is asleep).

Some embodiments of the implantable component 104C are configured to detect snoring by the recipient. For example, in some embodiments, the DSP 121 monitors measurements of the accelerometer/orientation sensor and compares those measurements to predefined patterns that indicate snoring. The DSP 121 then stores any indications of snoring in the memory 154. These indications are then uploaded via the coil 106 when the sound processor is attached to the recipient. The external component 102 is configured, in some embodiments, to generate an alert upon receiving an indication of sleep apnea from the implantable component 104B or implantable component 104C.

Note that while FIG. 1E illustrates an accelerometer/orientation sensor 152 and a temperature sensor 119 that are included as part of the implantable component 104C, in other embodiments, one or more of the accelerometer/orientation sensor 152 and/or temperature sensor 119 are disposed in the external component 102, or in a separate diagnostic tool.

Thus, FIGs. 1A-E illustrate example arrangements of an electro-acoustic hearing prosthesis. However, it is to be appreciated that the disclosed embodiments are implemented in a variety of electro-acoustic devices having alternative arrangements and/or in other types of hearing prostheses.

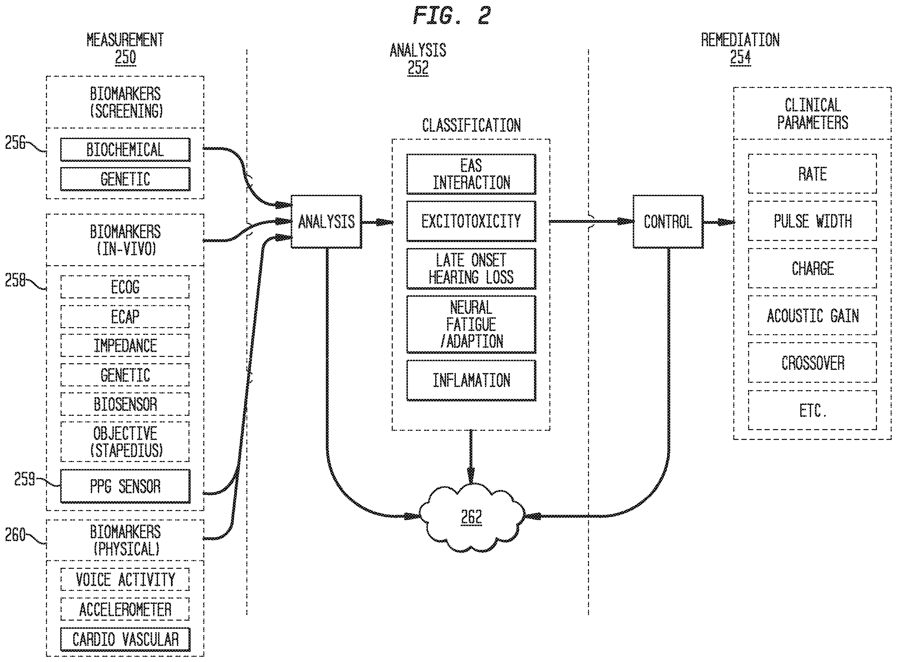

FIG. 2 is a schematic diagram illustrating techniques presented herein for in-situ proactive/predictive identification of cochlea health changes, for example, in the form of residual hearing loss by a recipient. The technique of FIG. 2 decreases the likelihood that the recipient of an electro-acoustic hearing prosthesis will develop a perceivable residual hearing loss (i.e., a residual hearing loss that is perceivable by the recipient). For ease of illustration, the example of FIG. 2 will be described with reference to the electro-acoustic hearing prosthesis 100A shown in FIG. -1B. However, the techniques described with respect to FIG. 2 are equally applicable to the embodiment of the electro-acoustic hearing prosthesis 100B shown in FIG. 1D, as well as other example arrangements.

Shown in FIG. 2 are three (3) general sections/stages, referred to as a measurement stage 250, an analysis stage 252, and a remediation stage 254. In the measurement stage 250, the electro-acoustic hearing prosthesis 100A, more specifically cochlea health analysis module 118, is configured to measure or otherwise determine/obtain one or more cochlea health biomarkers. As shown in FIG. 2, there are at least three different types of biomarkers that may be obtained by the cochlea health analysis module 118, including external or screening biomarkers 256, in-vivo biomarkers 258, and physical biomarkers 260. Screening biomarkers 256 are pre-determined biomarkers obtained through external processes, such as biochemical analyses, genetic screening, medical health records, and factors relating to hearing loss such as etiology and duration of deafness *etc.* These pre-determined biomarkers can be employed within analytical models to optimize the run time system. Here, the analytical model can, for example, segment or categorize the recipient based on established normative data. These categories can then decide the type of predictions and remedial actions may be taken by the live system (i.e., the hearing prosthesis operating in real-time). These remedial actions may, in certain arrangements, be tailored for high risk groups who are at a heightened danger of losing their residual hearing post operatively.

The physical biomarkers 260 are biomarkers that relate to one or more physical attributes. These physical attributes can include, for example, physical movements (e.g., detected by an inertial measurement unit 120), sound parameters (e.g., voice activity detections determined by sound processor 112), cardiovascular parameters (e.g., indicated by the PPG sensor 119A), *etc.* Distinct from the pre-determined biomarkers collected by medical health records, the physical attributes are inputs from the hearing prosthesis that are unrelated to physiological measurements from the cochlea or auditory pathway. Instead, the physical biomarkers 260 are associated with the recipient's movements, such as head movement and ambulatory motions. Some of the physical biomarkers 260 provide information characterizing, over time, the recipient's stability on their feet and head movements. Changes or particular patterns (pre-determined) of these physical attributes can provide predictions on whether the patient may be experiencing an onset or episode of vertigo or dizziness. These physical biomarkers 260 may be employed either alone or in conjunction with other biomarkers to predict the onset of an inner ear crisis.

Other physical biomarkers 260 may include monitored attributes of the recipient's own voice. Such monitoring can provide details on the vocal production of the individual, such as duration of utterance and conversational turns. As per the physical characterization, such information may yield, over time, changes to the recipient's hearing. For example, changes to the voiced production may indicate a change to the peripheral auditory pathway of the individual, reflecting a change to the inner ear and function. Again, these features may be either used independent or in conjunction with other inputs for automatic assessment of cochlea health.

Other physical biomarkers 260 include, in some embodiments, monitored attributes of the recipient's cardiovascular system. As discussed above, such monitoring can provide details on hypertension being experienced by the recipient, which, in some circumstances, leads to hearing impairment. Heart rate variability is indicative, in some circumstances, of a systemic infection. Such an infection presents a risk that the infection will propagate to the cochlea or vestibular system. This can result in permanent loss of either residual acoustic hearing or neural structures associated with electrical hearing. Cardiovascular biomarkers can also indicate a drop in blood pressure which, in some circumstances, indicates a dysfunctional vestibular system.

In some embodiments, the physical biomarkers 260 include biomarkers reported by the recipient. For example, a recipient may notice a change in their hearing ability and/or the performance of their cochlear implant. Some embodiments provide a user interface that allows a recipient to provide input indicating one or more biomarkers, such as indications of their hearing ability and/or cochlear implant performance over time. In some other embodiments, an own voice detection (OVD) algorithm is used to detect changes in performance of a hearing aid and/or cochlear implant. Some of these embodiments combine input from the OVD algorithm with monitoring of a sound environment and/or physical location of the recipient (e.g., via a global positioning system (GPS) receiver) to add strength to this detection system.

The in-vivo biomarkers 258 are biomarkers obtained by the cochlea health analysis module 118 through one or more in-situ measurements, such as cochlea response telemetry measurements, electrophysiological measurements, biochemical measurements, or cardiovascular measurements. Cochlea response telemetry measurements include those obtained from an electrocochleography (ECoG) sensor, or measurements including electrically evoked compound action potential (ECAP) measurements, higher evoked potentials measured from the brainstem and auditory cortex, and measurements of the electrical properties of the cochlea and the electrode array. Measurements of the electrical properties of the cochlea and the electrode array are generally and collectively referred to herein as "cochlea potential" measurements, and may include a number of voltage measurements, such as impedance measurements.

In addition, the hearing prosthesis may receive inputs from sensors, sometimes referred to herein as biosensors that are capable of extracting information relating to the homeostasis of the inner ear. The homeostasis of the inner ear refers to the constant equilibrium of the chemical environment in the inner ear. Maintenance of the homeostasis is critical to ensure the cells related to hearing function are protected and maintained. Examples of the types of biochemical feedback from these sensors might be the level of potassium and sodium levels in the perilymph in which the electrode array is immersed. Other chemical and biochemical measures may be taken to ascertain if homeostasis has been compromised or has altered since implantation of the cochlear implant.

Electrophysiological measurements and electrical properties refer to the use of the signals that are of biological origin or relate to the biological properties (electric potentials) as inputs to the analysis stage of the real-time algorithm. Each recipient may have, based on the pre-determined inputs, normal patterns or characteristic behaviors for each of these electrophysiological and cochlea potentials. These signals may be measured continuously during operation of the system and compared against these normative templates or patterns of normal behavior in an effort to determine whether the inner ear has altered in its health or function. Alternatively, individual or multiple inputs may be compared against each other or against prior time points to determine a similar analysis. The cochlea potentials may change in response to physiological changes that are independent from implant function, or as a direct consequence to either the acoustic and or electrical stimulus of the cochlea by the implant system.

An ECAP measurement refers to the capture of a set of electrical potentials generated in the recipient's cochlea 120 in response to the delivery of electrical stimulation to the cochlea. In contrast, an ECoG measurement refers to the capture of a set of electrical potentials generated in the recipient's cochlea 120 in response to the delivery of acoustic stimulation to the cochlea. The captured electrical potentials (i.e., a set of ECoG responses) may include a plurality of different stimulus related electrical potentials, such as the cochlear microphonic (CM), the cochlear summating potential (SP), the auditory nerve neurophonic (ANN), and the auditory nerve Action Potential (AP), which are measured independently or in various combinations. The cochlear microphonic is an alternating current (AC) voltage that mirrors the waveform of the acoustic stimulus at both low, moderate and high levels of acoustic stimulation. The cochlear microphonic is generated by the outer hair cells of the organ of Corti and is dependent on the proximity of the recording electrode(s) to the stimulated hair cells and the basilar membrane. In general, the cochlear microphonic is proportional to the displacement of the basilar membrane by the travelling wave phenomena.

The summating potential is the direct current (DC) response of the outer hair cells of the organ of Corti as they move in conjunction with the basilar membrane (i.e., reflects the time-displacement pattern of the cochlear partition in response to the stimulus envelope). The summating potential is the stimulus-related potential of the cochlea and can be seen as a DC (unidirectional) shift in the cochlear microphonic baseline. The direction of this shift (i.e., positive or negative) is dependent on a complex interaction between stimulus parameters and the location of the recording electrode(s).

The auditory nerve neurophonic is a signal recorded from the auditory nerve, while the auditory nerve Action Potential represents the summed response of the synchronous firing of the nerve fibers in response to the acoustic stimuli, and it appears as an alternating current voltage. The auditory nerve Action Potential is characterized by a series of brief, predominantly negative peaks, including a first negative peak (N1) and second negative peak (N2). The auditory nerve Action Potential also includes a magnitude and a latency. The magnitude of the auditory nerve Action Potential reflects the number of fibers that are firing, while the latency of the auditory nerve Action Potential is measured as the time between the onset and the first negative peak (N1).

In the example of FIGs. 1A-1C and FIG. 1D, an ECoG measurement and/or an ECAP measurement is initiated by the cochlea health analysis module 118. In the case of an ECoG measurement, the cochlea health analysis module 118 is configured to cause the receiver 142 to deliver acoustic stimulation to the recipient's cochlea 120 while, in the case of an ECAP measurement, the cochlea health analysis module 118 is configured to cause the stimulator unit 132 to deliver electrical stimulation to the recipient's cochlea 120. Following delivery of the acoustic or electrical stimulation, the cochlea analysis health module 118 is configured to receive a group/set of cochlea responses 170. As noted, the cochlea responses 170 (i.e., the ECoG or ECAP responses) are electrical potentials generated in the recipient's cochlea 120 when acoustic or electric stimulation are delivered to the cochlea 120. The cochlea responses 170 are obtained by one or more of the intra-cochlea electrodes 138 and are transmitted through the external coil 106 to the sound processor 112 and/or the cochlea health analysis module 118. In an embodiment, the cochlea responses 170 are transmitted from the external coil 106, through the sound processor 112, to the cochlea health analysis module 118. In another embodiment, the cochlea responses 370 are transmitted from the external coil 106 directly to the cochlea health analysis module 118.

The ECoG measurements and/or an ECAP measurements may be performed, for example, periodically, at preselected times, in response to user inputs, *etc.* In certain examples, the recipient may be provided with a notification indicating that measurements are about to be performed. Alternatively, the measurements may be conducted at sub-clinical levels that cannot be perceived by the user. The measurements may also be inter-dispersed in the clinical operation of the device such that the measures are not perceived and can be obtained continuously.

In accordance with certain embodiments presented herein, a hearing prosthesis is configured to analyze ambient sound signals received by the hearing prosthesis during normal operation (i.e., outside of a clinical setting) to identify portions of the sound signals that are sufficient to evoke an ECoG response from the cochlea. When a portion of a sound signal that is sufficient to evoke an ECoG response is identified, the hearing prosthesis itself performs an ECoG measurement using one or more implanted electrodes.

As noted, FIGs. 1A, 1B, and FIG. 2 illustrate the use of a receiver 142 to deliver acoustic stimulation for the performance of an ECoG measurement. However, embodiments of the present invention may be implemented in other hearing prostheses that deliver stimulation in a different manner to evoke an ECoG measurement (e.g., bone conduction devices or direct acoustic stimulators that deliver vibration to the cause movement of the cochlea fluid). It is also to be appreciated that some of the disclosed embodiments are implemented in hearing prostheses that do not deliver acoustic stimulation.

More specifically, as noted above, it is common for hearing prosthesis recipients to retain at least part of this normal hearing functionality (i.e., retain at least one residual hearing). Therefore, the cochlea of some hearing prosthesis recipient can be acoustically stimulated upon delivery of a sound signal to the recipient's outer ear without the aid of the hearing prosthesis itself (e.g., recipients who are implanted with only a cochlear implant). In such recipients, an ECoG measurement may be performed in response to the un-aided acoustic stimulation.

Returning to the specific example of FIG. 2, the analysis stage 252 involves the analysis and/or classification of the cochlea health biomarkers obtained during the measurement stage 250. In particular, the cochlea health analysis module 118 is configured to employ the real-time analysis of the cochlea health biomarkers (e.g., in-situ and/or external inputs) to determine, again in real-time, whether the recipient is likely to develop a perceivable residual hearing loss at some point in the future. Stated differently, the biomarkers are analyzed by a real-time algorithm to identify precursors to cochlea health changes (i.e., inner ear crises), such as future residual hearing loss (e.g., significant residual hearing loss, residual hearing loss that is noticeable/perceivable by the recipient, *etc.).*

The analysis of the various biomarkers to identify precursors to cochlea health changes may take a number of different forms. In one embodiment, the biomarkers are used to identify detectable events corresponding to patterns of behavior or established patterns relating to known pathologies, pathophysiology or physiology that correlate to potential future changes in the cochlea health, such as potential loss of residual hearing (i.e., predetermined risk factors for residual hearing loss) and or trauma to inner ear biological structures. Stated differently, the biomarkers may be used as inputs to a pattern matching algorithm that correlates various combinations of the biomarkers with patterns of behavior or established patterns relating to known pathologies, pathophysiology or physiology that correlate to inner ear crises. For example, the biomarkers may be analyzed to identify electro-acoustic phenomena within the recipient's cochlea that are suggestive of a cochlea health change, such as a potential residual hearing loss, and to classify the type or cause of the expected residual hearing loss. That is, the analysis of the morphology of various signals, possibly relative to each other, can identify/reveal events and/or patterns that occur even before a recipient's hearing changes (i.e., precursors to residual hearing changes). For example, an ECoG biomarker detected during measurement stage 250 may indicate that the stimulus input is at risk of causing excitotoxicity, which might contribute to either an acute or permanent loss of inner ear function that may manifest as a perceivable or non-perceivable residual hearing loss in the future. In that instance, the ECoG biomarker would be classified as related to excitotoxicity.

It is to be appreciated that the real-time algorithm may classify any number of biomarkers with different causes of perceivable residual hearing loss. For example, an ECoG biomarker may be classified as related to excitotoxicity, as well as to late onset hearing loss. In another example, an ECoG biomarker and an ECAP biomarker may both be classified as related to excitotoxicity.

FIG. 2 illustrates several possible "classifications" or "categories" of potential causes of perceivable residual hearing loss in recipients of electro-acoustic hearing prostheses for different biomarkers that may be determined and used by the cochlea health analysis module (i.e., the analysis state of the real-time algorithm executed by the hearing prosthesis). These classifications, which are further described below, include: (1) electroacoustic interaction, (2) excitotoxicity, (3) late onset hearing loss, and (4) neural fatigue or adaption. One skilled in the art will recognize that the causes described below are merely examples and that there are many causes and/or effects of perceivable residual hearing loss other than those provided herein. For example, in another embodiment the biomarkers may be used to classify dizziness or vertigo, which may be a result of residual hearing loss. In certain examples, the classifications may be utilized to "grade" the severity of the predicted hearing loss (e.g., low, medium, high). The assigned severity grade may be used to, for example, select the type of remedial action that is initiated, the timing of the remedial action, etc.

Referring first to electro-acoustic interaction, the concurrent provision of electrical and acoustic stimulation to the cochlea, be it aided or un-aided, can impair the transduction of an effective audibility of the residual acoustic hearing. This impairment relates to a reduction in quality of information (e.g., the magnitude and/or timing of a signal, *etc.)* presented to the acoustic structures. This particular impairment is driven by the interaction of the electrical stimulus via electrophonic effects or via recruitment of the neural structures through the spread of excitation. Either or both types of interaction may occur due to the wide spread of the electrical field associated with the delivery of electrical stimulus in commercial cochlear implant systems. The clinical impact of such interaction can include uncontrolled loudness, loss of timing information (e.g., localization or hearing in ambient noise), off-frequency hearing, and informational and energetic masking of information. Any of these impacts can lead to a loss in perceivable residual hearing (e.g., poorer clinical outcomes for the recipient).

Referring next to excitotoxicity, systems capable of delivering large amounts of charge, chronically, in the presence of either aided or un-aided acoustic hearing may pose a risk, either directly or indirectly, to the residual hearing or the overall cochlea health. In particular, the large spread of an applied electrical field and charge associated with electrical stimulation of the cochlea may chronically hyperpolarize the pre-synaptic acoustic hearing structures (including the synapse). If electrical stimulation is delivered chronically, this hyperpolarization may cause temporary or permanent damage to neural pathways, thereby causing a loss in perceivable residual hearing and or electrical hearing for the user.

Referring next to late onset hearing loss, following the surgical insertion of an electrode array into the cochlea, the structure is prone to additional physical, biological, acoustic, and/or electrical trauma. That is, a recipient's inner ear undergoes a number of significant mechanical, biological and biochemical events during surgical implantation, recovery, and during initiation and management of the clinical settings of the electro-acoustic hearing prosthesis. In the case of acoustic and electrical stimulation applied to the cochlea, there is a practical risk that the clinical stimulus, if set excessively, may trigger an immunological response from the cochlea through either direct or indirect mechanisms. Such an immunological response may lead to transient changes in the electrode impedance, corresponding changes in the vestibular system, and hydrops, *etc.* These changes may manifest clinically as dizziness, out-of-compliance states, fluctuating hearing levels, and temporary or permanent loss of residual hearing.

Referring next to neural fatigue/adaption, excessive delivery of electro-acoustic stimulation has been demonstrated to induce neural fatigue or long-term adaption in the neurophysiological structures of the cochlea. The onset of either or both of these phenomena may require device configuration changes or else impact the electro-acoustic hearing prosthesis if incoming stimuli are set at excessive levels. Neural fatigue here refers to either partial or complete conduction block of the neural signals evoked due to either acoustic or electrical stimulation, or a combination thereof. This blocking relates to the neurological mechanism of the nerve in that signals evoked at the periphery of the auditory nerve are either partially or fully blocked further up the pathway. In contrast, neural adaption relates to the innate regulatory system of the neurons whereby the size of the evoked response decreases or increases based on the type and duration of the stimulus input. For example, a prolonged stimulus input to the cochlea may decrease in perceived loudness due to the user. Both neural fatigue and adaption may be characterized for the user under normal operating conditions for the cochlear implant and then compared against prior time-points or patterns of pre-determined behavior associated with diagnosed cochlea health changes.

Furthermore, regarding dizziness/vertigo, it has been long established that recipients of cochlear implants may present with mild to severe episodes of dizziness or vertigo following implantation. The exact cause (pathology) of this remains somewhat unclear however it is thought, in some cases, to be a secondary effect of an underlying disruption to increased pressure within the endolymphatic space or an acute or chronic inflammatory response within the inner ear. Alternatively, the cause may relate to a blocked ductus reuniens.

As noted, in the analysis stage 252, the cochlea health analysis module 118 analyzes one or more of the above biomarkers in real-time to predict, and potentially classify, a recipient's cochlea health change, such as residual hearing loss. In certain embodiments, the cochlea health analysis module 118 is configured to collectively analyze a plurality of different objective inputs to predict changes to the recipient's residual hearing and/or other cochlea health changes. For example, the cochlea health analysis module 118 may be configured to analyze ECAP and/or ECoG biomarkers in combination with a cochlea potential measurement, such as an intra-cochlea impedance measurement, to predict an upcoming/future residual hearing loss.

The electrophysiological signals measured within the cochlea relate to the operation of various biological functions of hearing. In contrast the cochlea potentials (electrical properties) as measured by the voltage telemetry of the implantable component relates to the measurement of the electrode interface and surrounding tissue electrical properties of the biological media surrounding the electrode array. As the electrophysiological signals such as the ECoG are indicative of cell function, access to information pertaining to biological content of the media surrounding the array may be important in the diagnosis of cochlea health. This information may be either employed as an independent measure or in combination with the electrophysiological data in an effort to either improve the accuracy of diagnosis or detection, or to improve the sensitivity of the system to detecting the onset of crises within the cochlea such that remedial actions may be taken for maximum clinical efficacy. For example, changes to the biological content of the perilymph surrounding the electrode array may result from an inflammatory response that may be detected prior to the manifestation of electrophysiological changes within the cochlea. In this example, the altered biological content may cause a change to the bulk impedance between electrode contacts. Similarly, the electrical properties of the cochlea (cochlea potentials) may be monitored to better describe or diagnose changes observed in the electrophysiological signal measured from the cochlea. Here, the changes in the electrophysiological signal, in this working example, the ECoG, can be caused by one of many changes in the inner ear. To best diagnose the root cause of the change (and best treatment), the electrical properties may be analyzed, in this example, including the electrode voltage spread within the cochlea to determine the nature of the change more specifically. An example being the development of hydrops whereby an increase in the pressure of the scala media may cause a bulging of the basilar membrane and alter the relative position of the electrode array in the scala tympani, the position of which can be determined from the change in the electrical properties.

In the example of FIG. 2, the analysis stage 252 may further comprise storing data relating to the classification of the biomarkers in a computer-readable storage medium 262. The non-transitory computer-readable storage medium 262 may comprise one or more memories, and may be located on the electro-acoustic hearing prosthesis or remote from the electro-acoustic hearing prosthesis (e.g., the data may be stored via the Internet or the cloud).

As noted, the example of FIG. 2 illustrates that the techniques presented herein also utilize a remediation stage 254. The remediation stage 254 involves the initiation of one or more remedial actions in response to the identification of a residual hearing loss precursor (i.e., based on the analysis of the biomarkers during stage 252).

A number of different remedial actions may be initiated in accordance with the embodiments presented herein. For example, in certain embodiments a remedial action includes one or more adjustments to the operation of the electro-acoustic hearing prosthesis so as to proactively address the predicted changes in cochlea. That is, the results of the analysis stage 252 may be used to adjust one or more clinical parameters used by the electro-acoustic hearing prosthesis to generate the electro-acoustic signals in a manner that preserves the recipient's residual hearing (i.e., adjustments to electrical and/or acoustic clinical parameters). These adjustments can include adjustments configured to lessen the intensity and or overlap of the stimulation delivered to the cochlea (e.g., remove or minimize interactions between the electric and acoustic stimulation signals), prevent the incidence of excitotoxicity specific to cochlea sensory structures, minimize or mediate the onset of acoustic hearing loss (and in the event pharmacological agents are employed, optimizing the clinical parameters to complement drug therapy), or prevent the onset of neural fatigue or adaption. Adjustments to electrical clinical parameters to prevent cochlea health loss may also include adjustments to sequential clinical parameters (e.g. current level, pulse width, phase extender or third phase, rate or timing, mode, electrode, and frequency to electrode allocation), simultaneous clinical parameters (e.g., phased array parameters), and/or composite stimulation parameters. For example, the hearing prosthesis may reduce the instantaneous current level (CL) delivered to the cochlea by reducing the clinical current level on the electrode in question. In addition, as the charge of the stimulus is dictated by the current level and pulse width (PW), it may be possible to increase the pulse width and reduce the current level to maintain the same perceived loudness, but at the same time reduce the intensity of the stimulus as a remedial therapy to an identified cochlea health change. For stimulation overlap, the hearing prosthesis may either alter the frequency mapping of the acoustic and or electrical stimulus so that the stimulation is delivered to non-overlapping regions of the cochlea. This may be performed using imaging data of the electrode array insertion Adjustments to acoustic clinical parameters may include adjustments to, for example, applied frequencies (e.g., aidable frequencies and/or compressive frequency allocation), the crossover point/frequency (i.e., the frequency point or range indicating the areas/ranges that receive acoustic or electrical stimulation), sound intensity (e.g., gains, maximum power output, and/or maximum comfort level), and adaptive components (e.g., compression, such as onset, recovery, noise reduction, and/or directionality).

In summary of the above, the remedial actions initiated in response to the identification of a cochlea health change precursor are configured to counteract, terminate, or otherwise minimize the agitation/trigger of the cochlea health change.

The remediation stage 254 may also involve the administration of therapeutic substances (pharmacological agents) in response to an inflammatory response and or dizziness post-operatively (i.e., drug therapy). For example, the electro-acoustic hearing prostheses 100A and/or 100B may be configured to not only auto-regulate the clinical stimulus parameters (e.g., to determine if the symptoms abate), but also to administer regulated doses of pharmacological agents, such as corticosteroids. In certain circumstances, a remedial action can be the triggering of external alarms or delivery of communications to a caregiver via paired and/or connected communication devices.

In accordance with embodiments presented herein, once a remedial action is initiated, the cochlea health analysis module 118 is configured to monitor the efficacy of the remedial action. That is, the cochlea health analysis module 118 may continue to obtain and analyze one or more biomarkers, as described above, to ensure that the remedial action has achieved the underlying target effect (e.g., prevented the cochlea health change, slowed the cochlea health change, *etc.*)*.* Again, this decision may be based on any one more of the biomarkers described above If the remedial action has not achieved the target effect (i.e., the biomarkers indicate a potential for a cochlea health change), the cochlea health analysis module 118 may initiate one or more additional remedial actions. In certain examples, the failure to achieve a target effect may cause a shut-down of the hearing prosthesis, cause the hearing prosthesis to enter a "safe" mode of operation where, for example, only minimal or critical stimulation is presented to the recipient, or may cause the delivery of an alarm and/or the transmission communication to a caregiver, clinician, *etc.*

As noted, in certain embodiments, the analysis of the biomarkers includes a classification of the type and/or cause of the residual hearing loss. This classification may be advantageous to determine, for example, the appropriate remedial action (e.g., clinical treatment) to initiate within remediation stage 254. For example, the classification may be used to determine if a remedial action should be initiated immediately by the hearing prosthesis itself (e.g., adjust stimulation parameters) or if a more passive response (e.g., notification or a clinician or caregiver) is sufficient.

Provided below are several examples illustrating how the techniques presented herein may be utilized to proactively identify and prevent cochlea health losses. One skilled in the art will recognize that these following examples are illustrative applications of the techniques described herein.

### Example A: Excitotoxicity

In this example, the aim is to prevent excessive acoustic or electric stimulus of the cochlea from causing chronic depolarization of cochlea hearing structures, which may lead to progressive swelling of the synaptic connections to the neural elements, causing permanent damage to the auditory information pathway (or synapse). Thus, in this example, there is relatively little damage to the acoustic hearing structures before the synapse (i.e., the hair cells). Rather, damage primarily occurs to the post-synaptic structures (i.e., the neural elements used for electrical stimulation).

To reduce a potential for exitotoxicity, some embodiments reduce a level of stimulation based on a detected reduction in blood supply as indicated by the PPG sensor. While a cochlea at normal health with good blood supply can tolerate a nominal intensity and/or duration of stimulation, when blood supply is compromised, the ability of the cochlea to maintain a healthy status when being electrically stimulated can be reduced. Thus, restrictions on a maximum stimulation level (e.g. reduction in C-level from that determined in a normal programming session), or restrictions in a duration of stimulation (e.g. elapsed time of stimulation during a time period) may be appropriate. Under certain blood supply conditions, a complete cessation of stimulation may be appropriate. In some embodiments, the cochlear implant generates an alert indicating the recipient has entered a phase of compromised resilience to electrical stimulation.

### Example B: Tumor Growth

Some tumors are intralabyrinthine or present in an internal auditory canal (e.g., such as acoustic Neuromas). Other tumors include cholesteatomas (e.g. present in the middle ear or mastoid process). While intralabryinthine schwanomas are somewhat rare and generally do not present substantial risks, acoustic neuromas and cholesteotomas are relatively common. Input received from a PPG sensor can be used to identify these conditions. Some of the disclosed embodiments then generate an alert indicating that such a tumor may be present.

### Example C: Neural Fatigue/Adaption

In this example, the aim is to predict and manage the clinical efficacy of the delivered electro-acoustic stimulation. Acoustic signals alone can cause the outer hair cells to adapt, which is characterized by a drop in the cochlear microphonic and/or auditory nerve neurophonic peak-to-peak amplitude at a given frequency on the order of 15-20 milliseconds (lower frequencies apply to electro-acoustic signals). Electric signals alone can cause fatigue or adaption at the neural level (i.e., an increase in the neural thresholds), which is characterized by a decrease in the ECAP on the order of milliseconds or seconds in response to chronic delivery of electrical stimulation. Combined electro-acoustic stimulation can cause outer hair cell and/or neural fatigue or adaption.

A measurement of the cochlear microphonic and/or auditory nerve neurophonic can be made by applying an acoustic tonal input at lower frequencies. Any changes to the cochlear microphonic amplitude over the course of milliseconds are monitored and recorded. Input from a PPG sensor can be used to track trends in blood supply and if the recipient enters a period of weaker blood supply, a probability of neural fatigue is increased. In some cases, a compromised blood supply to the stria vascularis can impact an ability to manage potassium levels in the inner ear and maintain health of hair cells and synapses. An analysis determines whether the changes and/or computed probabilities are beyond a threshold. If so, a remedial action is provided by decreasing the acoustic input (e.g., by adjusting compression or gains) and re-measuring and monitoring the cochlear microphonic amplitude. For cases involving electrical stimulation, an identical process may be followed by measuring ECAP response instead of cochlear microphonic amplitude.

### Example D: Immunological Response

In this example, the aim is to predict the onset of an immunological response within the cochlea before permanent damage can occur to the cochlea hearing structures. An immune response may occur in response to an acute infection within the cochlea; this is characterized by a change in the electrical properties of the cochlea (e.g., impedance). An infection in the middle ear is relatively common and presents a risk of infection of the inner ear. In some circumstances, an infection begins in the mastoid cavity and then can spread further. In some cases, the pinnacle can be involved. Such an infection can then cause a fibrotic tissue reaction which fills the inner ear and results in conductive loss. Measurements obtained from the PPG sensor can indicate such a condition, for example, by showing increased demand and blood flow. As discussed above, some embodiments receive temperature information from an embedded temperature sensor 119C. The temperature information, in combination with input from the PPG sensor can provide indications of an infection. Some of the disclosed embodiments then generate an alert indicating that an infection may be present.

A measurement of the cochlear microphonic and/or auditory nerve neurophonic can be made by applying an acoustic tonal input at lower frequencies. A further measurement can be made of the electrical properties, such as the impedance. Any changes to the cochlear microphonic amplitude and/or electrical properties over the course of hours are monitored and recorded. An analysis determines whether a change is detected in both the cochlear microphonic and electrical properties, whether the changes are permanent (i.e., do not restore after stimulation ceases or drops due to a quiet environment), and whether the changes are beyond a threshold. If so, the electro-acoustic hearing prosthesis applies a remedial action comprising decreasing the acoustic input (e.g., by adjusting compression or gains) and re-measuring and monitoring the cochlear microphonic, auditory nerve neurophonic, and/or electrical properties, flagging an alarm, and administering drug therapy if the implant is equipped with a built-in pump.

Examples A-D, above, provide specific embodiments whereby changes to stimulation parameters may be made such that further deterioration of residual hearing can be prevented.

In an embodiment, the electro-acoustic hearing prosthesis has access to the computer-readable storage medium described above. The computer-readable storage medium may store data relating to other recipients' biomarkers and classifications and treatments thereof. Applying statistics to the data allows for the electro-acoustic hearing prosthesis to apply the most statistically valid treatment, thereby ensuring maximal clinical utility and allowing the treatment to adapt as the data grows and evolves.

As noted, it is well-established that recipients of electro-acoustic hearing prostheses may present with mild to severe episodes of dizziness or vertigo following implantation. While the exact cause of dizziness or vertigo remains somewhat unclear in this context, it is thought that, in some cases, dizziness or vertigo is a secondary effect of an underlying disruption to increased pressure within the endolymphatic space or an acute or chronic inflammatory response within the inner ear. Thus, the example of FIG. 2 may indirectly treat dizziness or vertigo by reducing the odds of a recipient developing a perceivable residual hearing loss.

Developments in electro-acoustic hearing prosthesis technology provide for chronic delivery of electrical and/or acoustic stimulation. Chronic stimulation can exacerbate the above-described causes of perceivable residual hearing loss. The example of FIG. 2 combats the negative effects of chronic stimulation by mitigating the possible residual hearing loss.

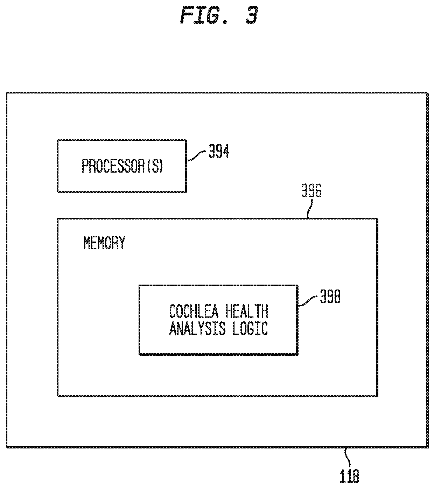

FIG. 3 is a schematic block diagram illustrating an arrangement for cochlea health analysis module 118 in accordance with an example embodiment. As shown, the hearing outcome tracking module 118 includes one or more hardware processors 394 (or other hardware processing circuitry) and a memory 396. The memory 396 includes cochlea health analysis logic 398.

The memory 396 may be read only memory (ROM), random access memory (RAM), or another type of physical/tangible memory storage device. Thus, in general, the memory 396 may comprise one or more tangible (non-transitory) computer readable storage media (e.g., a memory device) encoded with software comprising computer executable instructions and when the software is executed (by the one or more hardware processors 394) it is operable to perform the operations described herein with reference to cochlea health analysis module 118. In one example, memory 396 includes the computer readable medium 262 described above with reference to FIG. 2.

FIG. 3 illustrates a specific software implementation for cochlea health analysis module 118. However, it is to be appreciated that cochlea health analysis module 118 may have other arrangements. For example, cochlea health analysis module 118 may be partially or fully implemented with digital logic gates in one or more application-specific integrated circuits (ASICs). Alternatively, the one or more hardware processors 394 of hearing outcome tracking module may be the same or different processor as the sound processor 112 (FIGs. 1A-1D).

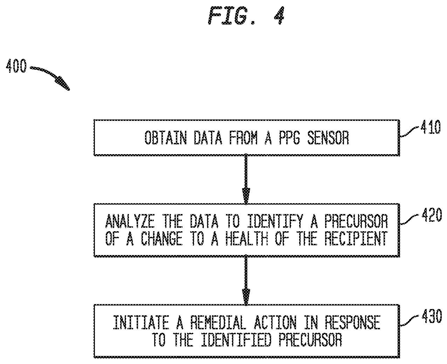

FIG. 4 is a flowchart illustrating an example method 400 in accordance with embodiments presented herein. Method 400 begins at operation 410 where an implantable medical device (e.g., cochlear implant, electro-acoustic hearing prosthesis, etc.) obtains a biomarker associated with a health of a recipient of the implantable medical device from a PPG sensor (e.g., obtains an input from a photoplethysmography (PPG) sensor). As discussed above with respect to FIGs. 1B and 1D, in some embodiments, the PPG sensor is positioned externally on the recipient's body. In some other embodiments the PPG sensor is an in-vivo sensor. For example, the PPG sensor is implanted in an ear canal of the recipient. Alternatively, the PPG sensor is implanted proximate to a blood line barrier of the cochlea.

At operation 420, the implantable medical device analyzes, in real-time, the biomarker (input) to identify a precursor of a change to the health of the recipient (e.g., a precursor of a change to the health of an inner ear of the recipient). For example, the biomarker indicates, in some embodiments, an inflammatory condition. As discussed above, data from a PPG sensor indicates, in various circumstances, cardiovascular condition(s) including one or more of blood flow to the cochlea, heart rate, cardiac cycle, respiration, blood oxygenation level, hypertension, heart rate variability, or a drop in blood pressure.

Some embodiments determine a confidence score of the determined precursor. In some embodiments, the precursor is identified based on data received from the PPG sensor. In some embodiments, the confidence score is further based on other information relating to a health of the recipient. For example, in some embodiments, the confidence score is based on clinical conditions such as a recipient's weight, age, or comorbidities. Some embodiments provide for manual entry of this information to enable confidence score determination based on other health information of the recipient.

A confidence of the identification is then evaluated against a criterion (e.g., a criterion that compares the confidence level to a predefined confidence threshold). If the evaluation indicates the confidence in the identification is sufficiently high (e.g. via the confidence level meeting one or more predefined criterion), some embodiments inhibit further evaluation or consideration of additional data from other sensors (e.g., the confidence in the identification of the precursor is sufficiently high to, for example, perform a remedial action based on the data received from the PPG sensor alone).

If the evaluation indicates the confidence in the identification of the precursor is not sufficiently high (e.g., less than a predefined threshold), some embodiments obtain data from one or more additional sensors, such as an ECoG sensor or an inertial measurement unit (e.g., indicating whether the recipient is experiencing challenges maintaining their balance). The confidence score of the identification of the precursor is then re-evaluated based on the input provided by the one or more additional sensors. If the confidence score is now sufficiently high, operation 420 is completed, at least in some embodiments. Otherwise, if the confidence score remains too low for further action, then no action is taken, at least in some embodiments.

Some embodiments consult a biomarker indicated by the PPG sensor in response to a confidence of an initial precursor determination, where in the initial precursor determination is made based on one or more other biomarkers (e.g. such as one or more of an ECOG, ECAP, or balance determination (e.g., via the IMU 120, in some embodiments, acting as a balance sensor). In some embodiments, a biomarker indicated by the PPG sensor is consulted when the initial precursor determination has an associated confidence score that is below a predefined confidence threshold. Thus, in these embodiments, one or more cardiovascular indications provided by the PPG sensor server to modify an existing confidence score associated with other biomarkers. If, upon consulting the PPG sensor, the confidence score sufficiently increases so as to transgress a predefined threshold, method 400 moves on to operation 430, where a remedial action is performed, as discussed further below.

At operation 430, in response to identification of a precursor of a change to the health, the implantable medical device initiates a remedial action. Remedial actions include, for example, one or more of stimulating a vestibular system of the recipient or adjusting an electrical stimulus regime of the cochlea by the implant. In some embodiments, the electro-acoustic hearing prosthesis generates an alert based on the identified precursor. For example, some embodiments of the electro-acoustic hearing prosthesis include a wireless interface that enables the prosthesis to communicate with a remote computer. The prosthesis communicates information relating to the precursor, and or indicates an alert to be generated. In some embodiments, the remote computer generates an alert via a variety of messaging technologies, such as email, text message, or other messaging application.

As described above, presented herein are techniques for monitoring biomarkers that relate to the cochlea health of a recipient of a hearing prosthesis in order to identify precursors to changes in the cochlea health. Given the transient and time dependent nature of the cochlea health changes, the hearing prosthesis may adapt one or more clinical parameters (e.g., acoustic and/or electrical stimulation parameters) to counter any observed changes considered to be deleterious to the residual hearing of the recipient.

As noted above, merely for ease of description, aspects of the techniques presented herein have been described with reference to an electro-acoustic hearing prosthesis. However, it is to be appreciated that the techniques presented herein may be used with other hearing devices/prostheses, including combinations of any of a cochlear implant, middle ear auditory prosthesis (middle ear implant), bone conduction device, direct acoustic stimulator, electro-acoustic prosthesis, auditory brain stimulators, *etc.* The techniques presented herein may also be used with systems that comprise or include tinnitus therapy devices, vestibular devices (e.g., vestibular implants), visual devices (i.e., bionic eyes), sensors, pacemakers, drug delivery systems, defibrillators, functional electrical stimulation devices, catheters, seizure devices (e.g., devices for monitoring and/or treating epileptic events), sleep apnea devices, electroporation devices, *etc.*

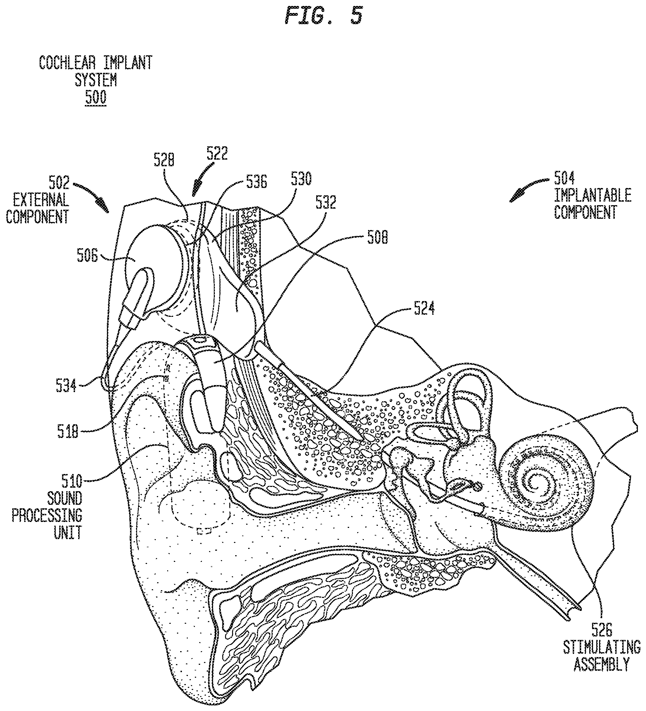

FIG. 5 is a schematic diagram of an illustrative cochlear implant system 500 configured to implement the cochlea health monitoring techniques presented herein. More specifically, FIG. 5 illustrates that the cochlear implant system 500 comprises an external component 502 and an internal/implantable component 504. The external component 502 is configured to be directly or indirectly attached to the body of a recipient, while the implantable component 504 is configured to be subcutaneously implanted within the recipient (i.e., under the skin/tissue of the recipient).

The external component 502 comprises an external coil 506 and a sound processing unit 510 connected via, for example, a cable 534. The external coil 506 is typically a wire antenna coil comprised of multiple turns of electrically insulated single-strand or multi-strand platinum or gold wire. Generally, a magnet (not shown in FIG. 5) is fixed relative to the external coil 506.

Similar to the embodiments of FIGs. 1A-1C, the sound processing unit 510 comprises one or more sound input elements, such as one or more microphones 508, a sound processor, an external transceiver unit (transceiver), a power source, and a cochlea health analysis module. The sound processing unit may be, for example, a behind-the-ear ("BTE") sound processing unit or other type of processing unit worn on the recipient's head.

The implantable component 504 comprises an implant body (main module) 522, a lead region 524, and an elongate intra-cochlear stimulating assembly (electrode array) 526. The implant body 522 generally comprises a hermetically-sealed housing 528 in which an internal transceiver unit (transceiver) 530 and a stimulator unit 532 are disposed. The implant body 522 also includes an internal/implantable coil 536 that is generally external to the housing 528, but which is connected to the transceiver 530 via a hermetic feedthrough (not shown). Implantable coil 536 is typically a wire antenna coil comprised of multiple turns of electrically insulated single-strand or multi-strand platinum or gold wire. The electrical insulation of implantable coil 536 is provided by a flexible molding (e.g., silicone molding), which is not shown in FIG. 5. Generally, a magnet (not shown in FIG. 1B) is fixed relative to the implantable coil 536.

Elongate stimulating assembly 526 is configured to be at least partially implanted in the recipient's cochlea 541 and includes a plurality of longitudinally spaced intra-cochlear stimulating contacts (e.g., electrical and/or optical contacts) that collectively form a contact array. Stimulating assembly 526 extends through an opening in the cochlea (e.g., cochleostomy, the round window, *etc.*) and has a proximal end connected to stimulator unit 532 via lead region 524 and a hermetic feedthrough (not shown in FIG. 5). As such, lead region 524 couples the stimulating assembly 526 to the implant body 522 and, more particularly, stimulator unit 532.

Returning to external component 502, the microphone(s) 508 and/or other sound input elements (not shown) are configured to detect/receive sound signals and generate electrical output signals therefrom. These output signals are representative of the detected sound signals. In addition to the one or more microphones 508, the sound processing unit 510 may include other types of sound input elements (e.g., telecoils, audio inputs, *etc.*) to receive sound signals. However, merely for ease of illustration, these other types of sound input elements have been omitted from FIG. 5.

The sound processor is configured execute sound processing and coding to convert the output signals received from the sound input elements into coded data signals that represent electrical stimulation for delivery to the recipient. That is, as noted, the cochlear implant system 500 operates to evoke perception by the recipient of sound signals received by the sound input elements (e.g., microphones 508) through the delivery of electrical stimulation signals and to the cochlear 541 of the recipient.

The coded signals are transcutaneously transferred to the implantable component 504 via external coil 506 and the implantable coil 536. In general, the coded data are received at the transceiver 530 and provided to the stimulator unit 532. The stimulator unit 532 is configured to utilize the coded signals to generate stimulation signals (e.g., current signals) for delivery to the recipient's cochlea via one or more stimulating contacts.

As noted above, a recipient's cochlea health (e.g., residual hearing, neural survival, *etc.*)*,* can change post-implantation. Therefore, in accordance with embodiments presented herein, the cochlear implant system 500 comprises a cochlea health analysis module 518 that may be substantially similar to cochlea health analysis module 118 of FIGs. 1A-1C. The cochlea health analysis module 518 is configured identify (i.e., predict) post-implantation changes to a recipient's cochlea health and, potentially, initiate one or more remedial actions to, for example, prevent significant changes to the recipient's cochlea health. More specifically, the cochlea health analysis module 518 is configured to measure, determine, or otherwise obtain one or more biomarkers associated with the recipient's cochlea health. For example, the cochlea health analysis module 118 is configured to obtain sensor data from a PPG sensor (not shown in FIG. 5), which can be positioned on or in the recipient 's body, and provides a biomarker indicating one or more of a heart rate, cardiac cycle, respiration, blood pressure, or blood oxygenation level of the recipient.

The cochlea health analysis module 518 is then configured to analyze one or more of these biomarkers to detect/identify one or more precursors of a change to the cochlea health (i.e., inner ear crises), such as precursors to residual hearing loss. Thus, in some embodiments, a sensor placed outside the inner ear (e.g., a PPG sensor placed on a body of the recipient) provides input useful, in combination with input from other sensors positioned within the inner year, in assessing cochlea health.

As noted above, precursors to cochlea health changes are detectable events corresponding to patterns of behavior or established patterns relating to known pathologies, pathophysiology or physiology that correlate to potential future changes in the cochlea health, such as potential loss of residual hearing (i.e., predetermined risk factors for residual hearing loss) and or trauma to inner ear biological structures. In one form, the precursors to cochlea health include inflammation or cardiovascular condition(s) that are detectable via the PPG sensor and/or individual changes in one or more of a heart rate, cardiac cycle, a respiration rate, blood pressure, or blood oxygenation level of the recipient.

Based on the analysis of the biomarkers and the identification of one or more cochlea health change precursors, the cochlea health analysis module 518 can initiate one or more actions (treatments) to remediate the cochlea health changes. In one example, the cochlea health analysis module 518 operates as a closed-loop element that is configured to auto-regulate, for example, the delivered stimulation in order to minimize the likelihood of damaging or masking the physiological structures supporting residual hearing (e.g., a real-time control / regulatory system to minimize the loss of residual hearing post-implantation). Without regulation of the delivered stimulation, there is a risk that the post-implantation residual hearing may be compromised by the inadequately configured stimulation. For example, the electrical stimulation could be improperly clinically configured so as to cause either acute or chronic changes (injurious) to the cochlea health or, to interfere with the transduction of information via the residual acoustic hearing structures.

Alternatively, changes to a recipient's health may arise after implantation that are indicative of changes to the recipient's cochlea health. For example, cardiovascular conditions indicated by the PPG sensor, in some cases, indicate blood flow to the cochlea and thereby impact a long term health of the organ. Hypertension detectable by the PPG sensor, in some circumstances, leads to hearing impairment. Heart rate variability detected by the PPG sensor indicates, in some circumstances, a systemic infection that risks propagation to the cochlea or vestibular system. Such an infection risks permanent loss of either residual acoustic hearing or neural structures associated with electrical hearing. A drop in blood pressure that is detected via the PPG sensor is indicative, in some circumstances, of a dysfunctional vestibular system.

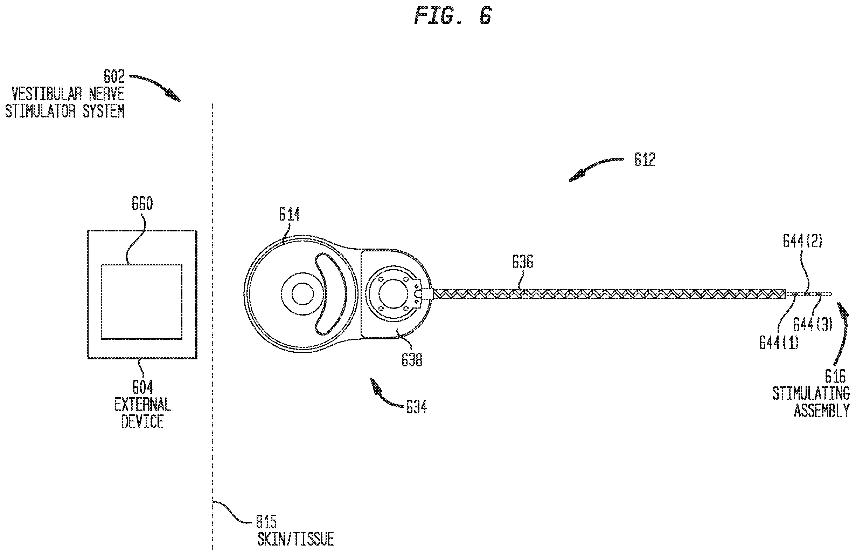

FIG. 6 illustrates an example vestibular stimulator system 602, with which embodiments presented herein can be implemented. As shown, the vestibular stimulator system 602 comprises an implantable component (vestibular stimulator) 612 and an external device/component 604 (e.g., external processing device, battery charger, remote control, *etc.).* The external device 604 comprises a wireless power transmitter unit 660. As such, the external device 604 is configured to transfer power (and potentially data) to the vestibular stimulator 612,

The vestibular stimulator 612 comprises an implant body (main module) 634, a lead region 636, and a stimulating assembly 616, all configured to be implanted under the skin/tissue (tissue) 615 of the recipient. The implant body 634 generally comprises a hermetically-sealed housing 638 in which RF interface circuitry, one or more rechargeable batteries, one or more processors, and a stimulator unit are disposed. The implant body 134 also includes an internal/implantable coil 614 that is generally external to the housing 638, but which is connected to the transceiver via a hermetic feedthrough (not shown).

The stimulating assembly 616 comprises a plurality of electrodes 644 disposed in a carrier member (e.g., a flexible silicone body). In this specific example, the stimulating assembly 616 comprises three (3) stimulation electrodes, referred to as stimulation electrodes 644(1), 644(2), and 644(3). The stimulation electrodes 644(1), 644(2), and 644(3) function as an electrical interface for delivery of electrical stimulation signals to the recipient's vestibular system.

The stimulating assembly 616 is configured such that a surgeon can implant the stimulating assembly adjacent the recipient's otolith organs via, for example, the recipient's oval window. It is to be appreciated that this specific embodiment with three stimulation electrodes is merely illustrative and that the techniques presented herein may be used with stimulating assemblies having different numbers of stimulation electrodes, stimulating assemblies having different lengths, *etc.*

As should be appreciated, while particular uses of the technology have been illustrated and discussed above, the disclosed technology can be used with a variety of devices in accordance with many examples of the technology. The above discussion is not meant to suggest that the disclosed technology is only suitable for implementation within systems akin to that illustrated in the figures. In general, additional configurations can be used to practice the processes and systems herein and/or some aspects described can be excluded without departing from the processes and systems disclosed herein.

This disclosure described some aspects of the present technology with reference to the accompanying drawings, in which only some of the possible aspects were shown. Other aspects can, however, be embodied in many different forms and should not be construed as limited to the aspects set forth herein. Rather, these aspects were provided so that this disclosure was thorough and complete and fully conveyed the scope of the possible aspects to those skilled in the art.

As should be appreciated, the various aspects (e.g., portions, components, etc.) described with respect to the figures herein are not intended to limit the systems and processes to the particular aspects described. Accordingly, additional configurations can be used to practice the methods and systems herein and/or some aspects described can be excluded without departing from the methods and systems disclosed herein.

Similarly, where steps of a process are disclosed, those steps are described for purposes of illustrating the present methods and systems and are not intended to limit the disclosure to a particular sequence of steps. For example, the steps can be performed in differing order, two or more steps can be performed concurrently, additional steps can be performed, and disclosed steps can be excluded without departing from the present disclosure. Further, the disclosed processes can be repeated.

Although specific aspects were described herein, the scope of the technology is not limited to those specific aspects. One skilled in the art will recognize other aspects or improvements that are within the scope of the present technology. Therefore, the specific structure, acts, or media are disclosed only as illustrative aspects. The scope of the technology is defined by the following claims.

## Claims

1. An implantable medical device (100A, 100B, 100C, 612), comprising:
a photoplethysmography (PPG) sensor (119A, 119B);
a stimulating assembly (526, 616) configured to be implanted in a recipient, the stimulating assembly (526, 616) comprising a plurality of stimulating contacts configured to deliver electrical stimulation signals to an inner ear of the recipient; and
a hardware processor (394) configured to:
obtain a cardiovascular parameter from the PPG sensor (119A, 119B);
identify a precursor of a change to a cochlea health of the recipient based on the cardiovascular parameter; and
initiate a remedial action in response to the identified precursor.

2. The implantable medical device (100A, 100B, 100C, 612) of claim 1, wherein the PPG sensor (119B) is implanted in an ear canal or at a blood-labyrinthine barrier (BLB) of the recipient, or wherein the PPG sensor (119B) is attached to a location on a body of the recipient that is external to an ear of the recipient.

3. The implantable medical device (100A, 100B, 100C, 612) of claim 1 or 2, wherein the hardware processor (394) is configured to:
determine a probability of an inflammatory condition of a middle ear or a probability of an infection of an inner ear of the recipient based on the cardiovascular parameter, wherein the initiating of the remedial action is based on the probability.

4. The implantable medical device (100A, 100B, 100C, 612) of claim 1 or 2, wherein the cardiovascular parameter indicates one or more of a heart rate, a cardiac cycle, a respiration, a blood pressure, or a blood oxygenation level of the recipient.

5. The implantable medical device (100A, 100B, 100C, 612) of claim 1 or 2, wherein the hardware processor (394) is configured to:
determine a confidence score based on the cardiovascular parameter; and
evaluate the confidence score against a predefined criterion, wherein the initiating of the remedial action is based on the evaluating.

6. The implantable medical device (100A, 100B, 100C, 612) of claim 5, wherein the hardware processor (394) is configured to:
inhibit further evaluation of other biomarkers based on the evaluation.

7. The implantable medical device (100A, 100B, 100C, 612) of claim 5, wherein the hardware processor (394) is configured to:
obtain additional data from an in-vivo sensor in response to the evaluating, wherein the determining of the confidence score is based on the additional data.

8. The implantable medical device (100A, 100B, 100C, 612) of claim 7, wherein the in-vivo sensor is at least one of an electrocochleography (ECoG) sensor or an inertial measurement unit.

9. The implantable medical device (100A, 100B, 100C, 612) of claim 1 or 2, wherein the hardware processor (394) is configured to:
determine a confidence score based on data obtained from an in-vivo sensor, wherein the data is obtained in response to the confidence score meeting a criterion.

10. The implantable medical device (100A, 100B, 100C) of claim 1 or 2, wherein the implantable medical device (100A, 100B, 100C) is a hearing prosthesis and the stimulating assembly (526) is configured to be implanted in an inner ear of the recipient to deliver stimulation to the inner ear of the recipient.

11. The implantable medical device (100A, 100B, 100C, 612) of claim 10, wherein to initiate a remedial action in response to the identified precursor, the hardware processor (394) is configured to:
adjust the stimulation delivered to the inner ear of the recipient;
in particular, wherein the implantable medical device (612) is a vestibular device, and wherein to adjust the stimulation delivered to the inner ear of the recipient, the hardware processor (394) is configured to adjust stimulation delivered to a vestibular system and/or a cochlea of the recipient.

12. The implantable medical device (100A, 100B, 100C, 612) of any one of the preceding claims, wherein the remedial action comprises generating, via a wireless interface of the implantable medical device (100A, 100B, 100C, 612), an alert.

13. The implantable medical device of any one of the preceding claims, wherein the implantable medical device (100A, 100B, 100C) is a hearing prosthesis comprising an in-vivo sensor, the in-vivo sensor comprising at least one of an electrocochleography (ECoG) sensor and an inertial measurement unit, and wherein the hardware processor (394) is further configured to obtain additional data from the in-vivo sensor and to identify the precursor of the change to the cochlea health of the recipient based on the cardiovascular parameter and the additional data.

14. A non-transitory computer readable storage medium comprising instructions that when executed by the hardware processor (394) of an implantable medical device (100A, 100B, 100C, 612) according to claim 1 cause the implantable medical device (100A, 100B, 100C, 612) to perform operations comprising:
obtaining, at the implantable medical device (100A, 100B, 100C, 612), input from a photoplethysmography (PPG) sensor (119A, 119B);
analyzing, at the implantable medical device (100A, 100B, 100C, 612), the input to identify a cardiovascular condition;
determining, based on the cardiovascular condition, a precursor of a change to a cochlea health of the recipient; and
initiating a remedial action at the implantable medical device (100A, 100B, 100C, 612) in response to the identified precursor.

15. A non-transitory computer-readable storage medium according to claim 14, further comprising instructions that when executed by the hardware processor (394) of the implantable medical device (100A, 100B, 100C, 612) cause the implantable medical device (100A, 100B, 100C, 612) to operate as an implantable medical device according to any one of claims 2 to 13.

## Patentansprüche

1. Implantierbare medizinische Vorrichtung (100A, 100B, 100C, 612), die umfasst:
einen PPG-Sensor (photoplethysmography sensor) (119A, 119B);
eine stimulierende Anordnung (526, 616), die zum Implantieren in einem Empfänger ausgeführt ist, wobei die stimulierende Anordnung (526, 660) eine Vielzahl stimulierender Kontakte umfasst, die so ausgeführt sind, dass sie einem Innenohr des Empfängers elektrische Stimulationssignale zuführen; sowie
einen Hardware Prozessor (394), der ausgeführt ist zum:
Beziehen eines kardiovaskulären Parameters von dem PPG-Sensor (119A, 119B);
Identifizieren einer Vorstufe einer Änderung einer Cochlea-Gesundheit des Empfängers auf Basis des kardiovaskulären Parameters; sowie
Einleiten eines Heilungsvorgangs in Reaktion auf die identifizierte Vorstufe.

2. Implantierbare medizinische Vorrichtung (100A, 100B, 100C, 612) nach Anspruch 1, wobei der PPG-Sensor (119B) in einem Gehörgang oder an einer Blut-Labyrinth-Schranke (BLB) des Empfängers implantiert wird, oder wobei der PPG-Sensor (119B) an einer Position an einem Körper des Empfängers angebracht wird, die sich außerhalb eines Ohrs des Empfängers befindet.

3. Implantierbare medizinische Vorrichtung (100A, 100B, 100C, 612) nach Anspruch 1 oder 2, wobei der Hardware-Prozessor (394) ausgeführt ist zum:
Bestimmen einer Wahrscheinlichkeit eines Entzündungszustandes eines Mittelohrs oder einer Wahrscheinlichkeit einer Infektion eines Innenohrs des Empfängers auf Basis des kardiovaskulären Parameters, wobei das Einleiten des Heilungsvorgangs auf der Wahrscheinlichkeit basiert.

4. Implantierbare medizinische Vorrichtung (100A, 100B, 100C, 612) nach Anspruch 1 oder 2, wobei der kardiovaskuläre Parameter eine Herzfrequenz, einen Herzzyklus, eine Atmung, einen Blutdruck oder/und einen Blutsauerstoffgehalt des Empfängers anzeigt.

5. Implantierbare medizinische Vorrichtung (100A, 100B, 100C, 612) nach Anspruch 1 oder 2, wobei der Hardware-Prozessor (394) ausgeführt ist zum:
Bestimmen eines Konfidenzwertes auf Basis des kardiovaskulären Parameters; und
Bewerten des Konfidenzwertes anhand eines vordefinierten Kriteriums, wobei das Einleiten des Heilungsvorgangs auf dem Bewerten basiert.

6. Implantierbare medizinische Vorrichtung (100A, 100B, 100C, 612) nach Anspruch 5, wobei der Hardware-Prozessor (394) ausgeführt ist zum:
Unterbinden weiterer Bewertung anderer Biomarker auf Basis der Bewertung.

7. Implantierbare medizinische Vorrichtung (100A, 100B, 100C, 612) nach Anspruch 5, wobei der Hardware-Prozessor (394) ausgeführt ist zum:
Beziehen zusätzlicher Daten von einem In-vivo-Sensor in Reaktion auf das Bewerten, wobei das Bestimmen des Konfidenzwertes auf den zusätzlichen Daten basiert.

8. Implantierbare medizinische Vorrichtung (100A, 100B, 100C, 612) nach Anspruch 7, wobei der In-vivo-Sensor ein ECoG-Sensor (elektrocochleography sensor) oder/und eine inertiale Messeinheit ist.

9. Implantierbare medizinische Vorrichtung (100A, 100B, 100C, 612) nach Anspruch 1 oder 2, wobei der Hardware-Prozessor (394) ausgeführt ist zum:
Bestimmen eines Konfidenzwertes auf Basis von einem In-vivo-Sensor bezogener Daten, wobei die Daten in Reaktion darauf bezogen werden, dass der Konfidenzwert ein Kriterium erfüllt.

10. Implantierbare medizinische Vorrichtung (100A, 100B, 100C) nach Anspruch 1 oder 2, wobei die implantierbare medizinische Vorrichtung (100A, 100B, 100C) eine Hörprothese ist und die stimulierende Anordnung (526) so ausgeführt ist, dass sie in ein Innenohr des Empfängers implantiert wird, um Stimulation des Innenohrs des Empfängers zu bewirken.

11. Implantierbare medizinische Vorrichtung (100A, 100B, 100C, 612) nach Anspruch 10, wobei der Hardware-Prozessor (394) so ausgeführt ist, dass er zum Einleiten eines Heilungsvorgangs in Reaktion auf die identifizierte Vorstufe durchführt:
Anpassen der an dem Innenohr des Empfängers bewirkten Stimulation;
wobei insbesondere die implantierbare medizinische Vorrichtung (612) eine vestibuläre Vorrichtung ist und wobei der Hardware-Prozessor (394) so ausgeführt ist, dass er, um die an dem Innenohr bewirkte Stimulation des Empfängers anzupassen, an einem vestibulären Systems und/oder einer Cochlea des Empfängers bewirkte Stimulation anpasst.

12. Implantierbare medizinische Vorrichtung (100A, 100B, 100C, 612) nach einem der vorangehenden Ansprüche, wobei der Heilungsvorgang umfasst, dass über eine drahtlose Schnittstelle der implantierbaren medizinischen Vorrichtung (100A, 100B, 100C, 612) eine Warnmeldung erzeugt wird.

13. Implantierbare medizinische Vorrichtung nach einem der vorangehenden Ansprüche, wobei die implantierbare medizinische Vorrichtung (100A, 100B, 100C) einen In-vivo- Sensor umfasst, der In-vivo-Sensor einen ECoG-Sensor oder/und eine inertiale Messeinheit umfasst und wobei der Hardware-Prozessor (394) des Weiteren so ausgeführt ist, dass er zusätzliche Daten von dem In-vivo-Sensor bezieht und die Vorstufe der Änderung der Cochlea-Gesundheit des Empfängers auf Basis des kardiovaskulären Parameters sowie der zusätzlichen Daten identifiziert.

14. Nichtflüchtiges computerlesbares Speichermedium, das Befehle umfasst, die, wenn sie durch den Hardware-Prozessor (394) einer implantierbaren medizinischen Vorrichtung (100A, 100B, 100C, 612) nach Anspruch 1 ausgeführt werden, die implantierbare medizinische Vorrichtung (100A, 100B, 100C, 612) veranlassen, Vorgänge durchzuführen, die umfassen:
Beziehen von Eingang von einem PPG-Sensor (119A, 119B) an der implantierbaren medizinischen Vorrichtung (100A, 100B, 100C, 612);
Analysieren des Eingangs an der implantierbaren medizinischen Vorrichtung (100A, 100B, 100C, 612), um einen kardiovaskulären Zustand zu identifizieren;
Bestimmen einer Vorstufe einer Änderung einer Cochlea-Gesundheit des Empfängers auf Basis des kardiovaskulären Zustandes; sowie
Einleiten eines Heilungsvorgangs an der implantierbaren medizinischen Vorrichtung (100A, 100B, 100C, 612) in Reaktion auf die identifizierte Vorstufe.

15. Nichtflüchtiges computerlesbares Speichermedium nach Anspruch 14, das des Weiteren Befehle umfasst, die, wenn sie durch den Hardware-Prozessor (394) der implantierbaren medizinischen Vorrichtung (100A, 100B, 100C, 612) ausgeführt werden, die implantierbare medizinische Vorrichtung (100A, 100B, 100C, 612) veranlassen, als eine implantierbare medizinische Vorrichtung nach einem der Ansprüche 2 bis 13 zu arbeiten.

## Revendications

1. Dispositif médical implantable (100A, 100B, 100C, 612), comprenant :
un capteur de photopléthysmographie (PPG) (119A, 119B) ;
un ensemble stimulant (526, 616) configuré pour être implanté chez un receveur, l'ensemble stimulant (526, 616) comprenant une pluralité de contacts stimulants configurés pour délivrer des signaux de stimulation électrique à une oreille interne du receveur ; et
un processeur matériel (394) configuré pour :
obtenir un paramètre cardiovasculaire du capteur de PPG (119A, 119B) ;
identifier un précurseur d'un changement à une santé de la cochlée du receveur basé sur le paramètre cardiovasculaire ; et
initier une mesure corrective en réponse au précurseur identifié.

2. Dispositif médical implantable (100A, 100B, 100C, 612) selon la revendication 1, dans lequel le capteur de PPG (119B) est implanté dans un canal auriculaire ou au niveau d'une barrière hémato-labyrinthique (BHL) du receveur, ou dans lequel le capteur de PPG (119B) est fixé à un emplacement sur un corps du receveur qui est externe à une oreille du receveur.

3. Dispositif médical implantable (100A, 100B, 100C, 612) selon la revendication 1 ou 2, dans lequel le processeur matériel (394) est configuré pour :
déterminer une probabilité d'un état inflammatoire d'une oreille médiane ou une probabilité d'une infection d'une oreille interne du receveur sur la base du paramètre cardiovasculaire, dans lequel l'initiation de la mesure corrective est basée sur la probabilité.

4. Dispositif médical implantable (100A, 100B, 100C, 612) selon la revendication 1 ou 2, dans lequel le paramètre cardiovasculaire indique l'un ou plusieurs d'une fréquence cardiaque, d'un cycle cardiaque, d'une respiration, d'une tension artérielle, ou d'un niveau d'oxygénation du sang du receveur.

5. Dispositif médical implantable (100A, 100B, 100C, 612) selon la revendication 1 ou 2, dans lequel le processeur matériel (394) est configuré pour :
déterminer un indice de confiance basé sur le paramètre cardiovasculaire ; et
pour évaluer l'indice de confiance par rapport à un critère prédéfini, dans lequel l'initiation de la mesure corrective est basée sur l'évaluation.

6. Dispositif médical implantable (100A, 100B, 100C, 612) selon la revendication 5, dans lequel le processeur matériel (394) est configuré pour :
inhiber une évaluation supplémentaire d'autres biomarqueurs basés sur l'évaluation.

7. Dispositif médical implantable (100A, 100B, 100C, 612) selon la revendication 5, dans lequel le processeur matériel (394) est configuré pour :
obtenir des données additionnelles de la part d'un capteur *in-vivo* en réponse à l'évaluation, dans lequel la détermination de l'indice de confiance est basée sur les données additionnelles.

8. Dispositif médical implantable (100A, 100B, 100C, 612) selon la revendication 7, dans lequel le capteur *in-vivo* est au moins l'un d'un capteur d'électrocochléographie (ECoG) ou d'une unité de mesure inertielle.

9. Dispositif médical implantable (100A, 100B, 100C, 612) selon la revendication 1 ou 2, dans lequel le processeur matériel (394) est configuré pour :
déterminer un indice de confiance basé sur les données obtenues de la part d'un capteur *in-vivo,* dans lequel les données sont obtenues en réponse à l'indice de confiance satisfaisant un critère.

10. Dispositif médical implantable (100A, 100B, 100C) selon la revendication 1 ou 2, dans lequel le dispositif médical implantable (100A, 100B, 100C) est une prothèse auditive et l'ensemble stimulant (526) est configuré pour être implanté dans une oreille interne du receveur pour délivrer une stimulation à l'oreille interne du receveur.

11. Dispositif médical implantable (100A, 100B, 100C, 612) selon la revendication 10, dans lequel pour initier une mesure corrective en réponse au précurseur identifié, le processeur matériel (394) est configuré pour :
ajuster la stimulation délivrée à l'oreille interne du receveur ;
en particulier, dans lequel le dispositif médical implantable (612) est un dispositif vestibulaire, et dans lequel pour ajuster la stimulation délivrée à l'oreille interne du receveur, le processeur matériel (394) est configuré pour ajuster une stimulation délivrée à un système vestibulaire et/ou à une cochlée du receveur.

12. Dispositif médical implantable (100A, 100B, 100C, 612) selon l'une quelconque des revendications précédentes, dans lequel la mesure corrective comprend la génération, par l'intermédiaire d'une interface sans fil du dispositif médical implantable (100A, 100B, 100C, 612), d'une alerte.

13. Dispositif médical implantable selon l'une quelconque des revendications précédentes, dans lequel le dispositif médical implantable (100A, 100B, 100C) est une prothèse auditive comprenant un capteur *in-vivo,* le capteur *in-vivo* comprenant au moins l'un d'un capteur d'électrocochléographie (ECoG) et d'une unité de mesure inertielle, et dans lequel le processeur matériel (394) est en outre configuré pour obtenir des données additionnelles de la part du capteur *in-vivo* et pour identifier le précurseur du changement à la santé de la cochlée du receveur sur la base du paramètre cardiovasculaire et des données additionnelles.

14. Support de stockage non transitoire lisible par ordinateur comprenant des instructions qui lorsqu'exécutées par le processeur matériel (394) d'un dispositif médical implantable (100A, 100B, 100C, 612) selon la revendication 1 amènent le dispositif médical implantable (100A, 100B, 100C, 612) à exécuter des opérations comprenant les étapes consistant à :
obtenir, au niveau du dispositif médical implantable (100A, 100B, 100C, 612), une entrée provenant d'un capteur de photopléthysmographie (PPG) (119A, 119B) ;
analyser, au niveau du dispositif médical implantable (100A, 100B, 100C, 612), l'entrée pour identifier un état cardiovasculaire ;
déterminer, sur la base de l'état cardiovasculaire, un précurseur d'un changement à une santé de la cochlée du receveur ; et
à initier une mesure corrective au niveau du dispositif médical implantable (100A, 100B, 100C, 612) en réponse au précurseur identifié.

15. Support de stockage non transitoire lisible par ordinateur selon la revendication 14, comprenant en outre des instructions qui lorsqu'exécutées par le processeur matériel (394) du dispositif médical implantable (100A, 100B, 100C, 612) amènent le dispositif médical implantable (100A, 100B, 100C, 612) à fonctionner comme un dispositif médical implantable selon l'une quelconque des revendications 2 à 13.
